# EUROPEAN PATENT SPECIFICATION

(11) **EP 4 469 042 B1**
(45) Date of publication and mention of the grant of the patent: **26.08.2026**
(21) Application number: 23701732.2
(22) Date of filing: 25.01.2023
(51) Int. Cl.: A61K 31/404, A61K 31/4709, A61K 31/7105, A61K 31/713, A61K 31/135, A61K 45/06, C12N 15/113, A61P 25/24, A61P 25/18

(54) **A GLUN2D INHIBITOR FOR USE IN THE TREATMENT OR RELAPSE PREVENTION OF A DEPRESSIVE EPISODE**
GLUN2D-INHIBITOR ZUR VERWENDUNG BEI DER BEHANDLUNG ODER RÜCKFALLPRÄVENTION EINER DEPRESSIVEN EPISODE
INHIBITEUR DE GLUN2D DESTINÉ À ÊTRE UTILISÉ DANS LE TRAITEMENT OU LA PRÉVENTION DES RECHUTES D'UN ÉPISODE DÉPRESSIF

(30) Priority: 25.01.2022 EP 22153076
(43) Date of publication of application: 04.12.2024
(73) Proprietor: Albert-Ludwigs-Universität Freiburg, 79098 Freiburg (DE)
(72) Inventor: NORMANN, Claus, 79117 Freiburg (DE); VESTRING, Stefan, 79104 Freiburg (DE)
(74) Representative: Maiwald GmbH
(86) International application number: PCT/EP2023/051726
(87) International publication number: WO 2023/144163

(56) References cited:
- WO-A1-2020/069934
- US-A1- 2014 275 529
- US-A1- 2016 368 897
- US-A1- 2021 017 149
- US-A1- 2021 361 631
- IKEDA K ET AL: "Reduced spontaneous activity of mice defective in the @e4 subunit of the NMDA receptor channel", MOLECULAR BRAIN RESEARCH, ELSEVIER SCIENCE BV, AMSTERDAM, NL, vol. 33, no. 1, 1 October 1995 (1995-10-01), pages 61 - 71, XP027443615, ISSN: 0169-328X, [retrieved on 19951001], DOI: 10.1016/0169-328X(95)00107-4
- SWANGER SHARON A. ET AL: "A Novel Negative Allosteric Modulator Selective for GluN2C/2D-Containing NMDA Receptors Inhibits Synaptic Transmission in Hippocampal Interneurons", ACS CHEMICAL NEUROSCIENCE, vol. 9, no. 2, 2 November 2017 (2017-11-02), US, pages 306 - 319, XP055930578, ISSN: 1948-7193, DOI: 10.1021/acschemneuro.7b00329
- EAPEN ALEN V ET AL: "Multiple roles of GluN2D-containing NMDA receptors in short-term potentiation and long-term potentiation in mouse hippocampal slices", NEUROPHARMACOLOGY, ELSEVIER, AMSTERDAM, NL, vol. 201, 9 October 2021 (2021-10-09), XP086852661, ISSN: 0028-3908, [retrieved on 20211009], DOI: 10.1016/J.NEUROPHARM.2021.108833
- HENRY J.M. FERGUSON ET AL: "Glutamate dependent NMDA receptor 2D is a novel angiogenic tumour endothelial marker in colorectal cancer", ONCOTARGET, vol. 7, no. 15, 12 April 2016 (2016-04-12), XP055592731, DOI: 10.18632/oncotarget.7812
- YAMAMOTO HIDEKO ET AL: "Loss of GluN2D subunit results in social recognition deficit, social stress, 5-HT2Creceptor dysfunction, and anhedonia in mice", NEUROPHARMACOLOGY, vol. 112, 30 July 2016 (2016-07-30), pages 188 - 197, XP029780348, ISSN: 0028-3908, DOI: 10.1016/J.NEUROPHARM.2016.07.036
- ZHANG BING ET AL: "Ketamine activated glutamatergic neurotransmission by GABAergic disinhibition in the medial prefrontal cortex", NEUROPHARMACOLOGY, ELSEVIER, AMSTERDAM, NL, vol. 194, 2 November 2020 (2020-11-02), XP086635970, ISSN: 0028-3908, [retrieved on 20201102], DOI: 10.1016/J.NEUROPHARM.2020.108382
- IDE SOICHIRO ET AL: "Role of NMDA receptor GluN2D subunit in the antidepressant effects of enantiomers of ketamine", vol. 135, no. 3, 1 November 2017 (2017-11-01), JP, pages 138 - 140, XP055930029, ISSN: 1347-8613, Retrieved from the Internet <URL:https://www.sciencedirect.com/science/article/pii/S134786131730186X/pdfft?md5=b6cf6c65873c42797e9da5fb65f48d8a&pid=1-s2.0-S134786131730186X-main.pdf> DOI: 10.1016/j.jphs.2017.11.001
- VESTRING STEFAN ET AL: "Animal Models of Depression - Chronic Despair Model (CDM)", no. 175, 23 September 2021 (2021-09-23), XP055929588, Retrieved from the Internet <URL:http://dx.doi.org/10.3791/62579> DOI: 10.3791/62579

## Description

### FIELD OF THE INVENTION

The present invention relates to a GluN2D inhibitor or a mGluR2 inhibitor for use in the treatment or relapse prevention of a depressive episode. Vectors encoding some of these inhibitors are also provided for use in the relapse prevention or treatment of a depressive episode. Also provided are pharmaceutical compositions comprising these inhibitors, preferably for use in the treatment or relapse prevention of a depressive episode.

### BACKGROUND OF THE INVENTION

Psychiatry is a medical specialty that is concerned with diagnosis, prevention, and treatment of mental disorders that are related to mood, behavior, cognition and perceptions.

Depressive episodes characterize (and occur as part of) a number of such disorders, as defined in ICD-10: F31-F34. One of these disorders is Major Depressive Disorder (MDD). MDD is characterized by depressed mood, diminished interest in pleasure, insomnia, psychomotor agitation or retardation, loss of energy, difficulties in concentration and in many cases recurrent thought of death or suicide. It can present as a single episode or, typically, as a recurring disorder with relapses over lifetime. Single episodes of MDD persist for more than two weeks, but sometimes for years and cause significant distress or impairment in social, occupational or other important areas of functioning. MDD afflicts anywhere from 10 to 20% of the population and causes enormous socioeconomic damage due to direct (medical and psychological treatment, hospitalization) or indirect (loss of productivity, occupational disability) costs. According to the World Health Organization, MDD is by far the leading cause of life years lost to disability worldwide. Moreover, MDD is a major cause of death by suicide. In the United States, MDD is a contributing cause to the majority of the approximately 30,000 annual deaths by suicide. It has additionally been speculated that some unknown proportion of the 100,000 deaths by other unnatural means such as motor vehicle accidents, homicide and workplace accidents are also related to underlying depressive symptoms. Such deaths are the sixth leading cause of mortality in the United States.

Medical treatment of depression over the years has included the use of psychotherapy and prescription antidepressive drugs. In many metaanalyses examining the efficacy of antidepressive drugs, an adequate course of antidepressant treatment over several weeks leads to a remission in one third of patients, a partial improvement of depressive symptoms in another third, and a non-response in the remaining patients. Around one third of all MDD patients develop treatment resistance, defined as non-response to two or more consecutive medical treatment trials. For unknown reasons, most antidepressant drugs exhibit a latency of 4-6 weeks after the initiation of treatment until an antidepressive effect becomes obvious. Moreover, antidepressant drugs produce side effects in many patients, such as nausea, sexual dysfunction, cognitive slowing, emotional dulling, lethargy, and sleep disturbances, as well as potentially dangerous interactions with other medications. More recently, an association has been noted between the use of prescription antidepressants and the emergence of suicidal ideation, which is observed in a previously non-suicidal population. This risk appears particularly prominent in younger patients, e.g., those under the age of 24. This has in turn led to resistance to the use of this class of medication in pediatric, adolescent, and post-adolescent populations. Somewhat ironically, such under-treatment may have been associated with a spike in suicide deaths in the under-19 population between 2003 and 2004. Antidepressant drugs are used both in the acute treatment of depressive episodes and for relapse prevention, especially in patients which have experienced multiple and/or very severe episodes in their lifetime.

In the last few years, the non-competitive N-methyl-D-aspartate receptor (NMDAR) antagonist R,S-ketamine, originally used as an anesthetic drug, has been introduced as a rapid-acting antidepressant. It reduces symptoms of anhedonia, depressed mood and suicidal ideation within hours upon administration in a greater percentage of patients than standard antidepressive drugs, but care has to be taken to administer this active within the appropriate dosing window as higher doses result in undesired anaesthetic effects. Known side effects of R,S-ketamine at sub-anesthetic doses include dissociative symptoms, anxiety and a rise of blood pressure, limiting widespread clinical use. Moreover, treatment regimens aiming at sustained response or relapse prevention with R,S-ketamine are currently unclear. In addition, the widespread use of R,S-ketamine as a drug of abuse due to its intoxicating effects has provoked skepticisms regarding a medical use in psychiatry.

US 2021/0017149 A1 discloses a series of optimization of GluN2D-selective antagonists of the NMDA receptors with DQP scaffold, see paragraph [0209]. US 2021/0017149 A1 further discloses in paragraph [0075] that in one embodiment the compounds disclosed therein are used in a method of treatment or prophylaxis of a number of diseases including, amongst others, depression. Importantly, US 2021/0017149 A1 fails to disclose any experimental data that the compounds disclosed therein can in fact be used for treating depression such that an effective treatment of depression is not disclosed therein.

US 2016/0368897 A1 discloses a class of compounds as selective inhibitors of the GluN2C-and GluN2D-containing NMDA receptors, see paragraph [0290]. US 2016/0368897 A1 further discloses in claim 15 that a pharmaceutical composition comprising a compound of claim 1 is used in a method of treating or preventing a number of diseases including, amongst others, depression. Importantly, US 2016/0368897 A1 fails to disclose any experimental data that the compounds disclosed therein can in fact be used for treating depression such that an effective treatment of depression is not disclosed therein. US 2016/368897 A1 also fails to make an effective treatment of depression plausible because the reference cited in US 2016/3688897 A1 in this respect, Traynelis et al., fails to disclose any connection between GluN2C/D and depression.

WO 2020/069934 A1 discloses QNZ-46, which is characterized as GluN2C/D subunit-containing NMDA glutamate receptor antagonist, see page 3, line 11. In a further aspect, WO 2020/069934 A1 discloses on page 6, lines 4 and 5 that a composition including a compound as disclosed therein can be used in the treatment or prophylaxis of disorders or diseases of the nervous system involving myelin pathology, wherein, amongst others, depression is listed on page 6, lines 9 and 10 as other neurological disease that involves significant myelin damage to which the invention according to WO 2020/069934 A1 may be applicable (see also page 1, lines 18 to 20 of WO 2020/069934 A1, where depression is, amongst others, disclosed as other neurological disease affecting grey matter but having an important white matter component). Importantly, WO 2020/069934 A1 fails to disclose any experimental data that the compounds disclosed therein can in fact be used for treating depression such that an effective treatment of depression is not disclosed therein. Moreover, WO 2020/069934 A1 fails to disclose any experimental data or rationale or a citation that would make it plausible that depression is a neurological disease that involves significant myelin damage or has an important white matter component. To the contrary, the skilled person is not aware of any link between depression and myelin damage / white matter.

WO 2010/088408 discloses *inter alia* compounds and methods of treating or preventing disorders associated with NMDA receptor activity, wherein, amongst others, depression is listed, see e.g. the abstract of WO 2010/088408. Importantly, WO 2010/088408 fails to disclose any experimental data that the compounds disclosed therein can in fact be used for treating depression such that an effective treatment of depression is not disclosed therein. It seems that Preskorn et al. is referred to in WO 2010/088408 when it comes to depression, see page 4, second paragraph of WO 2010/088408. Preskorn et al., however, discloses that inhibiting GluN2B will cause cognitive disruption and psychotomimetic effects similar to those produced by ketamine in several patients.

Summarizing the above, an effective treatment of depression by the compounds targeting the NMDAR as disclosed in US 2021/0017149 A1, US 2016/0368897 A1, WO 2020/069934 A1 and WO 2010/088408 has not been shown. Such an effective treatment has also not been made plausible by the disclosure of these prior art documents or the prior art documents referred to therein in this respect.

Considering in particular US 2021/0017149 A1 and the GluN2D-selective antagonists of the NMDAR disclosed therein, it is noteworthy that US 2021/0017149 A1 not only fails to disclose any data that GluN2D-selective antagonists would be useful for treating depression, i.e. that GluN2D would need to be inactive when aiming at treating depression, but that there is even prior art that discloses that active GluN2D seems to be required for treating depression, namely in disclosing that active GluN2D seems to be necessary for the antidepressive effects of R,S-ketamine and R-ketamine, see Zhang et al. and Ide et al.

There have also been suggestions that GluN2B, another subunit of NMDAR, may play a role in depression and could potentially be targeted for treatment, but several patients in studies to this end experienced cognitive disruption and psychotomimetic effects similar to those produced by ketamine, see Hansen et al.; Preskorn et al.

Given the high prevalence and severity of MDD, there is an urgent need to provide further actives for treating this important disorder. Ideally, novel actives a) should be more effective in a higher percentage of patients than standard antidepressive drugs; b) should produce fewer side effects by increased specificity to better defined targets; and/or c) should exhibit a rapid onset of action and a sustained action.

### OBJECTS AND SUMMARY OF THE INVENTION

The inventors of the present invention have surprisingly identified the GluN2D subunit of the N-methyl-D-aspartate receptor (NMDAR) and the presynaptic metabotropic glutamate receptor 2 (mGluR2) as targets for the treatment and relapse prevention of a depressive episode, preferably a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34.

While it is known that R,S-ketamine can be administered for the treatment of a depressive episode and while this activity is assumed to be linked to R,S-ketamine's antagonistic activity towards NMDAR, it could be shown herein for the first time that it is the subunit GluN2D of the NMDAR that is responsible for the observed effect. This opens up a completely new field of specifically targeting this particular subunit, i.e. GluN2D, when aiming for the treatment of a depressive episode. As shown by data herein, different GluN2D inhibitors including both small molecule inhibitors, e.g. NAB-14, as well as siRNA (see Example 3), are capable of achieving the desired effect on the treatment of a depressive episode. Such inhibitors will, however, not result in undesired anaesthetic or dissociative effects as they specifically target GluN2D and not the NMDAR as such which is also implicated in anaesthesia and dissociation, as is the case for R,S-ketamine at higher doses, and be effective in a higher percentage of patients than standard antidepressive drugs, as is observed with R,S-ketamine.

The inventors also found that the desired effect can be achieved when targeting mGluR2. This was again shown by using a small molecule inhibitor (see Example 4).

GluN2D subunits of NMDARs are almost exclusively expressed on inhibiting interneurons and thereby regulate the inhibition of the activity of postsynaptic cells by GABAergic mechanisms. Therefore, inhibition of GluN2D disinhibits postsynaptic cells, synaptic activity and synaptic plasticity. On the other hand, mGluR2 is predominantly expressed in presynaptic neurons and functionally acts as an inhibiting autoreceptor, i.e. activation of mGluR2 inhibits the activity of presynaptic neurons, synaptic transmission and synaptic plasticity. Therefore, inhibition of mGluR2 increases the activity of presynaptic neurons, synaptic activity and synaptic plasticity. Both mechanisms (inhibition of GluN2D and mGluR2) therefore result in increased neuronal activity and, ultimately, network function in the brain; therefore, both can be conceptualized as disinhibition by different cellular mechanisms. In addition to directly modulating synaptic transmission, such mechanisms increase synaptic plasticity.

Synaptic plasticity controls how effectively two neurons communicate with each other. It is defined as the capability of the brain to functionally and, at later stages, morphologically adapt to external stimuli and is regarded as the molecular correlate of learning and memory. Mechanisms which increase synaptic transmission, glutamate exocytosis, or activity of NMDAR on postsynaptic cells increase synaptic plasticity. Therefore, disinhibition (i.e. by inhibition of GluN2D or mGluR2) positively modulate synaptic plasticity.

Both disinhibition and increased synaptic plasticity represent key factors in the treatment response for antidepressants in general, including for example R,S-ketamine (see Castrén; Castrén and Antila). Accordingly, inhibition of either GluN2D or mGluR2 leads to these same antidepressant effects but potentially avoids many commonly observed side effects of other antidepressants.

In a **first aspect,** the present invention is directed to a GluN2D inhibitor (which may also be referred to as inhibitor of the GluN2D subunit of the N-methyl-D-aspartate receptor [NMDAR]) for use in the treatment or relapse prevention of a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34, wherein the GluN2D inhibitor is not R,S-ketamine or a pharmaceutically acceptable salt thereof, S-ketamine or a pharmaceutically acceptable salt thereof, or R-ketamine or a pharmaceutically acceptable salt thereof.

In an embodiment, the GluN2D inhibitor causes disinhibition and/or increases synaptic plasticity. In an embodiment, disinhibition and/or increase of synaptic plasticity is the result of GluN2D inhibition leading to inhibition of inhibitory interneurons on which GluN2D is expressed. This in turn breaks the feedback loop between postsynaptic cells and these interneurons (see also Fig-ure 5).

In another embodiment, the GluN2D inhibitor is selected from the group consisting of a small molecule targeting GluN2D, an antibody directed to GluN2D or an antigen-binding fragment thereof, an antisense oligonucleotide targeting the GluN2D mRNA, a small interfering RNA (siRNA) targeting the GluN2D mRNA, a short hairpin RNA (shRNA) targeting the GluN2D mRNA, a microRNA (miRNA) targeting the GluN2D mRNA and a CRISPR-guide RNA (sgRNA) targeting GluN2D gene transcription.

In a preferred embodiment, the GluN2D inhibitor is a small molecule targeting GluN2D, wherein the small molecule may e.g. be a compound of formula (I) or (III):

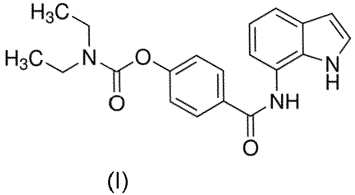

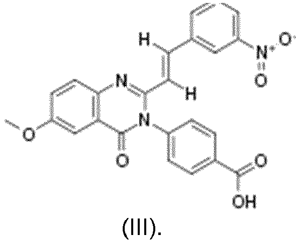

In a preferred embodiment, the GluN2D inhibitor is an antisense oligonucleotide targeting the GluN2D mRNA, wherein the antisense oligonucleotide is capable of binding to and/or is at least partially complementary to a region of the GRIN2D gene or a regulatory element, preferably at least one regulatory element in the gene's close vicinity.

In another preferred embodiment, the GluN2D-inhibitor is a siRNA targeting the GluN2D mRNA, wherein the siRNA is capable of interfering with the gene expression of the GluN2D gene and comprises a first strand at least partially complementary to 15 nucleotides of the GluN2D gene, and a second strand of 15 to 30 nucleotides in length, wherein at least 12 nucleotides of the first strand and second strand are complementary to each other and form a siRNA duplex. As an example, the first strand has the sequence of SEQ ID NO: 1 while the second strand has the sequence of SEQ ID NO: 2.

In another preferred embodiment, the GluN2D-inhibitor is a sgRNA targeting GluN2D gene transcription, wherein the sgRNA is at least partially complementary to 15 nucleotides of the GluN2D gene or its upstream regulatory elements, and wherein in addition a CRISPR protein lacking endonuclease activity is administered, preferably the CRISPR protein is fused to at least a domain of Krüppel associated box (KRAB) protein.

In a particularly preferred embodiment, the depressive episode is selected from the group consisting of a depressive episode occurring in isolation and a depressive episode occurring during bipolar affective disorder, recurrent depressive disorder, persistent mood disorder and major depressive disorder (MDD) including subtypes of MDD, such as in particular the subtypes melancholic depression, atypical depression, catatonic depression, postpartum depression and seasonal affective disorder. The treatment and relapse prevention of a depressive disorder occurring during bipolar effective disorder and MDD is in particular preferred in the present aspect.

As a depressive episode is a major symptom of the afore-mentioned diseases, the first aspect may alternatively be formulated in an embodiment as the use in the treatment or relapse prevention of the diseases themselves, i.e. as for use in the treatment or relapse prevention of bipolar affective disorder, recurrent depressive disorder, persistent mood disorder and major depressive disorder (MDD) including subtypes of MDD, such as in particular the subtypes melancholic depression, atypical depression, catatonic depression, postpartum depression and seasonal affective disorder. The treatment and relapse prevention of MDD and bipolar affective disorder is in particular preferred in the present aspect.

In yet another preferred embodiment of the first aspect, a GluN2D inhibitor is for use in the treatment of a depressive episode or the above diseases, respectively.

The first aspect of the present invention may alternatively be expressed as follows: A method of treating or preventing a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34, the method comprising administering to a subject in need thereof a GluN2D inhibitor, wherein the GluN2D inhibitor is not R,S-ketamine or a pharmaceutically acceptable salt thereof, S-ketamine or a pharmaceutically acceptable salt thereof, or R-ketamine or a pharmaceutically acceptable salt thereof. All embodiments of the first aspect as outlined above of course also apply for this alternative expression of the subject matter as method of treatment.

In a **second** aspect, the present invention is directed to a mGluR2 inhibitor (which may also be referred to as inhibitor of the glutamate receptor 2) for use in the treatment or relapse prevention of a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34, wherein the mGluR2 inhibitor is not 2R,6R-Hydroxynorketamine (HNK) or a pharmaceutically acceptable salt thereof.

In an embodiment thereof, the mGluR2 inhibitor causes disinhibition and/or increases synaptic plasticity. In an embodiment, disinhibition and/or increase of synaptic plasticity is the result of mGluR2 inhibition leading to inhibition of autoinhibition of presynaptic cells on which mGluR2 is expressed. This breaks an autoregulatory inhibition loop in presynaptic cells.

In another embodiment, the mGluR2 inhibitor is selected from the group consisting of a small molecule targeting mGluR2, an antibody directed to mGluR2 or an antigen-binding fragment thereof, an antisense oligonucleotide targeting mGluR2 mRNA, a small interfering RNA (siRNA) targeting mGluR2 mRNA, a short hairpin RNA (shRNA) targeting mGluR2 mRNA, a microRNA (miRNA) targeting mGluR2 mRNA and a CRISPR-guide RNA (sgRNA) targeting mGluR2 gene transcription.

In a preferred embodiment, the mGluR2 inhibitor is a small molecule targeting mGluR2, wherein the small molecule may e.g. be a compound of formula (II):

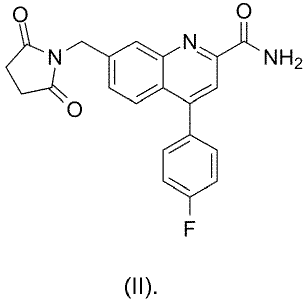

In a preferred embodiment, the mGluR2 inhibitor is an antisense oligonucleotide targeting the mGluR2 mRNA, wherein the antisense oligonucleotide is capable of binding to and/or is at least partially complementary to a region of the mGluR2 gene or regulatory elements, preferably at least one regulatory element in the gene's close vicinity.

In another preferred embodiment, the mGluR2 inhibitor is a siRNA targeting the mGluR2 mRNA, wherein the siRNA is capable of interfering with the gene expression of the mGluR2 gene and comprises a first strand at least partially complementary to 15 nucleotides of the mGluR2 gene, and a second strand of 15 to 30 nucleotides in length, wherein at least 12 nucleotides of the first strand and second strand are complementary to each other and form a siRNA duplex. As an example, the first strand has the sequence of SEQ ID NO: 3 while the second strand has the sequence of SEQ ID NO: 4.

In another preferred embodiment, the mGluR2 inhibitor is a sgRNA targeting mGluR2 gene transcription, wherein the sgRNA is at least partially complementary to 15 nucleotides of the mGluR2 gene or its upstream regulatory elements, and wherein in addition a CRISPR protein lacking endonuclease activity is administered, preferably the CRISPR protein is fused to at least a domain of Krüppel associated box (KRAB) protein.

In a particularly preferred embodiment, the depressive episode is selected from the group consisting of a depressive episode occurring in isolation and a depressive episode occurring during bipolar affective disorder, recurrent depressive disorder, persistent mood disorder and major depressive disorder (MDD) including subtypes of MDD, such as in particular the subtypes melancholic depression, atypical depression, catatonic depression, postpartum depression and seasonal affective disorder. The treatment or relapse prevention of a depressive disorder occurring during MDD or bipolar affective disorder is in particular preferred in the present aspect.

As a depressive episode is a major symptom of the afore-mentioned diseases, the second aspect may alternatively be formulated in an embodiment as the use in the treatment or relapse prevention of the diseases themselves, i.e. as for use in the treatment or relapse prevention of bipolar affective disorder, recurrent depressive disorder, persistent mood disorder and major depressive disorder (MDD) including subtypes of MDD, such as in particular the subtypes melancholic depression, atypical depression, catatonic depression, postpartum depression and seasonal affective disorder. The treatment or relapse prevention of MDD and bipolar affective disorder is in particular preferred in the present aspect.

In yet another preferred embodiment of the second aspect, a mGluR2 inhibitor is for use in the treatment of a depressive episode or the above diseases, respectively.

The second aspect of the present invention may alternatively be expressed as follows: A method of treating or preventing a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34, the method comprising administering to a subject in need thereof a mGluR2 inhibitor, wherein the mGluR2 inhibitor is not 2R,6R-Hydroxynorketamine (HNK) or a pharmaceutically acceptable salt thereof. All embodiments of the first aspect as outlined above of course also apply for this alternative expression of the subject matter as method of treatment

In a **third aspect,** the present invention is directed to a vector encoding (i) a GluN2D inhibitor selected from the group consisting of an antisense oligonucleotide targeting the GluN2D mRNA, a siRNA targeting the GluN2D mRNA, a shRNA targeting the GluN2D mRNA, a miRNA targeting the GluN2D mRNA and a sgRNA targeting GluN2D gene transcription or (ii) a mGluR2 inhibitor selected from the group consisting of an antisense oligonucleotide targeting the mGluR2 mRNA, a siRNA targeting the mGluR2 mRNA, a shRNA targeting the mGluR2 mRNA, a miRNA targeting the mGluR2 mRNA and a sgRNA targeting mGluR2 gene transcription for use in the treatment or relapse prevention of a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34.

The embodiments relating to the specific inhibitors as well as the depressive episode and diseases comprising a depressive episode as major symptom as outlined above for the first and second aspects apply for the third aspect as well.

The third aspect of the present invention may alternatively be expressed as follows: A method of treating or preventing a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34, the method comprising administering to a subject in need thereof a vector (i) a GluN2D inhibitor selected from the group consisting of an antisense oligonucleotide targeting the GluN2D mRNA, a siRNA targeting the GluN2D mRNA, a shRNA targeting the GluN2D mRNA, a miRNA targeting the GluN2D mRNA and a sgRNA targeting GluN2D gene transcription or (ii) a mGluR2 inhibitor selected from the group consisting of an antisense oligonucleotide targeting the mGluR2 mRNA, a siRNA targeting the mGluR2 mRNA, a shRNA targeting the mGluR2 mRNA, a miRNA targeting the mGluR2 mRNA and a sgRNA targeting mGluR2 gene transcription.

In a **fourth aspect,** the present invention is directed to a pharmaceutical composition comprising a GluN2D inhibitor, wherein the GluN2D inhibitor is not R,S-ketamine or a pharmaceutically acceptable salt thereof, S-ketamine or a pharmaceutically acceptable salt thereof, or R-ketamine or a pharmaceutically acceptable salt thereof, or a mGluR2 inhibitor, wherein the mGluR2 inhibitor is not 2R,6R-Hydroxynorketamine (HNK) or a pharmaceutically acceptable salt thereof, and a pharmaceutically acceptable excipient, preferably for use in the treatment or relapse prevention of a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34. In an embodiment of the fourth aspect, the GluN2D inhibitor is further not a compound of formula (I):

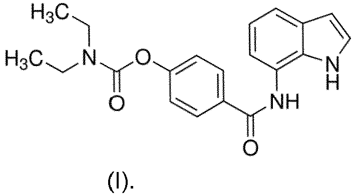

In an embodiment of the fourth aspect, the GluN2D inhibitor is selected from the group consisting of a small molecule targeting GluN2D, an antibody directed to GluN2D or an antigen-binding fragment thereof, an antisense oligonucleotide targeting the GluN2D mRNA, a small interfering RNA (siRNA) targeting the GluN2D mRNA, a short hairpin RNA (shRNA) targeting the GluN2D mRNA, a microRNA (miRNA) targeting the GluN2D mRNA and a CRISPR-guide RNA (sgRNA) targeting GluN2D gene transcription. In this embodiment, the small molecule targeting GluN2D may not be a compound of formula (I):

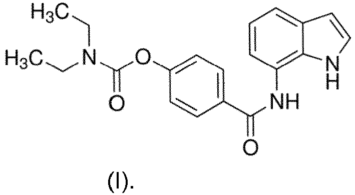

In an embodiment of the fourth aspect, the GluN2D inhibitor is selected from the group consisting of an antisense oligonucleotide targeting the GluN2D mRNA, a siRNA targeting the GluN2D mRNA, a shRNA targeting the GluN2D mRNA, a miRNA targeting the GluN2D mRNA and a sgRNA targeting GluN2D gene transcription, and the pharmaceutically acceptable excipient comprises a lipid.

In yet another embodiment of the fourth aspect, the mGluR2 inhibitor is selected from the group consisting of a small molecule targeting mGLuR2, an antibody directed to mGluR2 or an antigen-binding fragment thereof, an antisense oligonucleotide targeting the mGluR2 mRNA, a small interfering RNA (siRNA) targeting the mGluR2 mRNA, a short hairpin RNA (shRNA) targeting the mGluR2 mRNA, a microRNA (miRNA) targeting the mGluR2 mRNA and a CRISPR-guide RNA (sgRNA) targeting mGluR2 gene transcription.

In yet another embodiment of the fourth aspect, the mGluR2 inhibitor is selected from the group consisting of an antisense oligonucleotide targeting the mGluR2 mRNA, a siRNA targeting the mGluR2 mRNA, a shRNA targeting the mGluR2 mRNA, a miRNA targeting the mGluR2 mRNA and a sgRNA targeting mGluR2 gene transcription; and the pharmaceutically acceptable excipient comprises a lipid.

In a preferred embodiment of the fourth aspect, the GluN2D inhibitor is a siRNA targeting the GluN2D mRNA, the pharmaceutically acceptable excipient comprises at least two lipids and the pharmaceutical composition is a lipid formulation, preferably an LNP formulation.

In yet another preferred embodiment of the fourth aspect, the mGluR2 inhibitor is a siRNA targeting the mGluR2 mRNA, the pharmaceutically acceptable excipient comprises at least two lipids and the pharmaceutical composition is a lipid formulation, preferably an LNP formulation.

### DESCRIPTION OF THE FIGURES

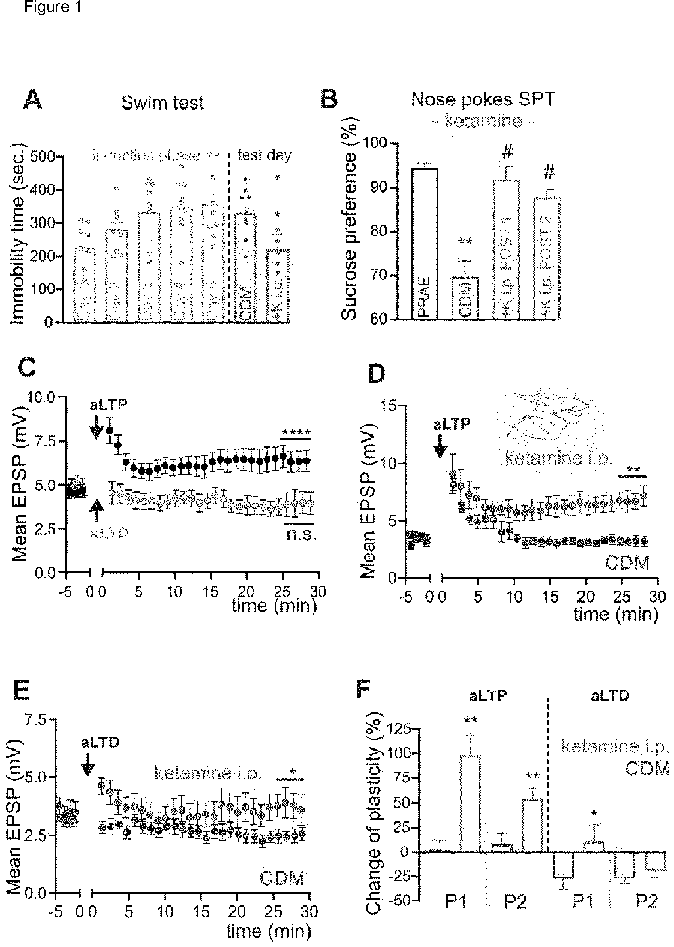

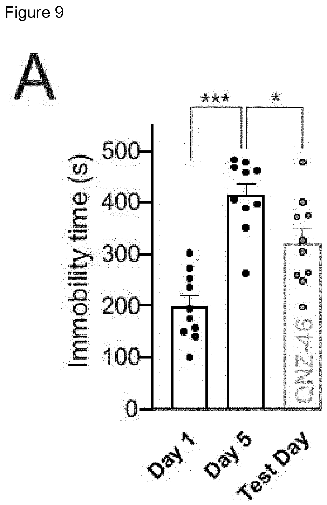

**Figure 1** - **R,S-Ketamine rehabilitates stress-induced changes of synaptic plasticity.**
   **A** Mice were forced to swim for five consecutive days; induction phase. During this induction phase, immobility time was recorded in each swim session, showed a steady increase from day 1 to day 5 and remained stable on the test day, 2 days after the induction phase, in the control condition (CDM). Previous injection of 10 mg/kg R,S-ketamine significantly reduced immobility time on the test day (Ki.p.). **B** Sucrose preference (measured by nose-pokes-sucrose-preference test) was significantly reduced in CDM (CDM vs. PARE), but could be completely reestablished by R,S-ketamine injection, measured at POST1 (first night after the injection) and POST 2 (second night after the injection) (CDM vs. POST1 and CDM vs. POST2). **C** After a stable baseline (-5 to 0 min), aLTP stimulation resulted in a stable and significant increase of mean EPSP amplitude (25 to 30 min), whilst aLTD did not result in a significant change of mean EPSP amplitude. (Each dot represents the means of ten EPSP per protocol). **D** CDM application resulted in a complete blockade of LTP inducibility, whilst a single R,S-ketamine injection was capable of completely reestablishing stress-induced changes (min 25 to 30; CDM vs. CDM + R,S-ketamine i.p.). E CDM facilitated LTD induction, resulting in a significant decrease of EPSP amplitude, whilst ketamine injection reversed this inducibility (min 25 to 30; CDM vs. CDM + R,S-ketamine i.p.). F Overview of the changes of plasticity induced by aLTP and aLTD protocols in CDM and CDM treated with R,S-ketamine. P1= protocol 1; CDM induction and afterwards R,S-ketamine application, P2 = protocol2; R,S-ketamine injection before CDM induction. Note that R,S-ketamine mainly exerts its effects via reestablishment of LTP. Data are means ±SEM. *P <.05, #P <.05 **P <.01, ****P <.0001.

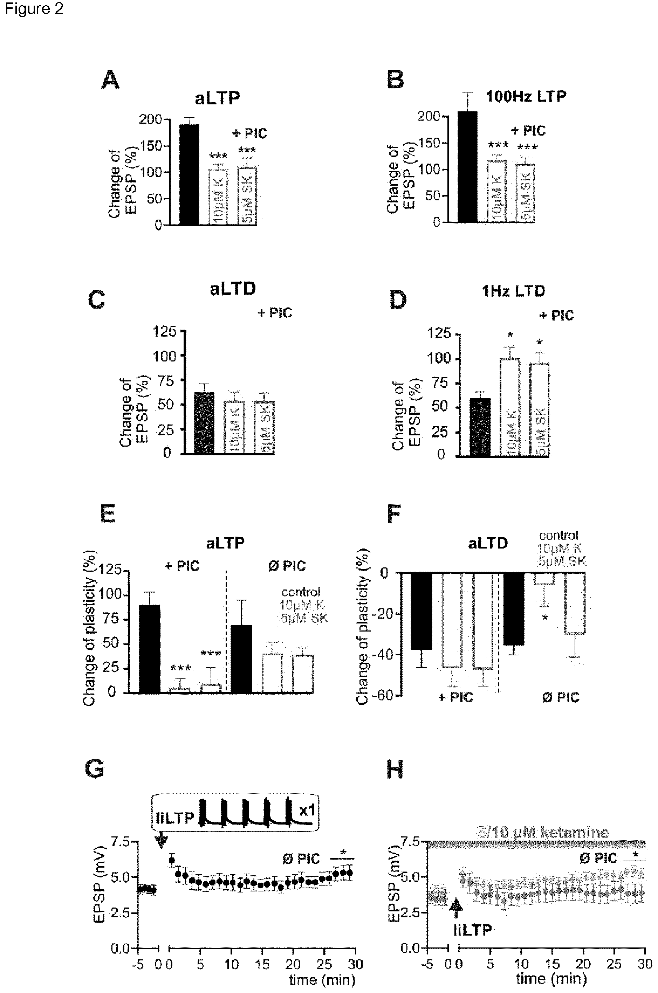

**Figure 2** **- Interneurons play key role in the modulation of plasticity by R,S-ketamine**
   **A/B** Application of R,S-ketamine (10 µM) or S-ketamine (5 µM) in the bathing solution of brain slice resulted in a significant blockade of inducibility of LTP in CA3-CA1 synapse by an associative LTP protocol (aLTP; A) and an non-associative LTP Protocol (100HZ LTP; B). C/D Application of neither R,S-ketamine (10 µM) nor S-ketamine (5 µM) had an effect on EPSP amplitude after an associative LTD protocol (C), whilst LTD inducibility via an non-associative LTD protocol (1Hz LTD) was abolished in the presence of R,S-ketamine or S-ketamine (D). **E/F** Overview of modulation of LTP(E) and LTD(F) inducibility by R,S-ketamine and S-ketamine in the presence and absence of Picrotoxin (PIC). Note that LTP is not affected by R,S-ketamine and S-ketamine if PIC is absent, but significantly blocked if PIC is present (E). **G** Low intensity LTP (liLTP) is able to significantly increase mean EPSP amplitude in the absence of PIC (baseline -5 to 0 min vs. 25 to 30min). **H** Different dosages of ketamine (5/10 µM) affect the inducibility of liLTP differently. Whilst a higher dosage (10 µM) resulted in a blockade of LTP induction, a lower dosage (5 µM) resulted in an even enhanced LTP induction. Data are means ±SEM. *P <.05, ***P <.001.

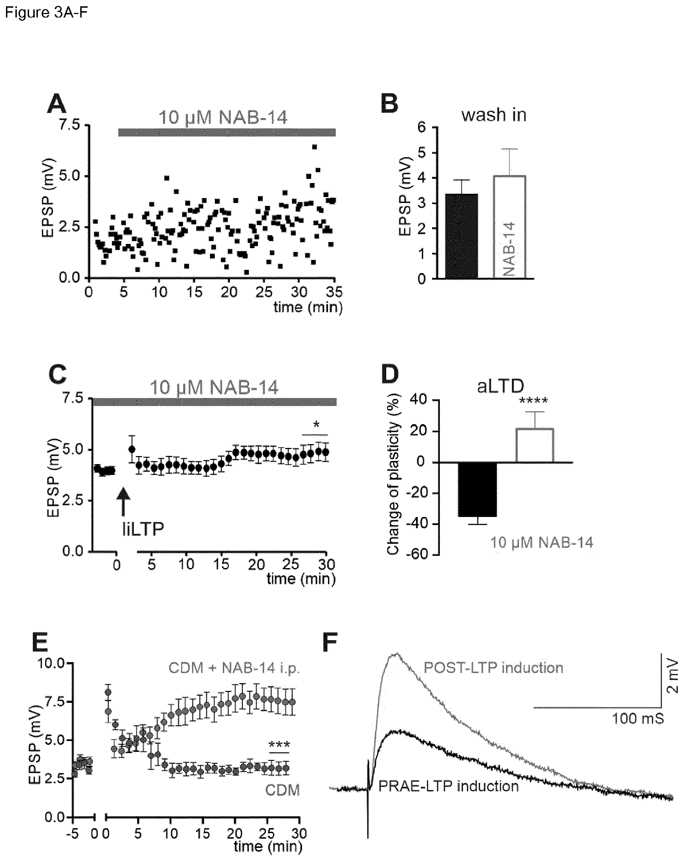

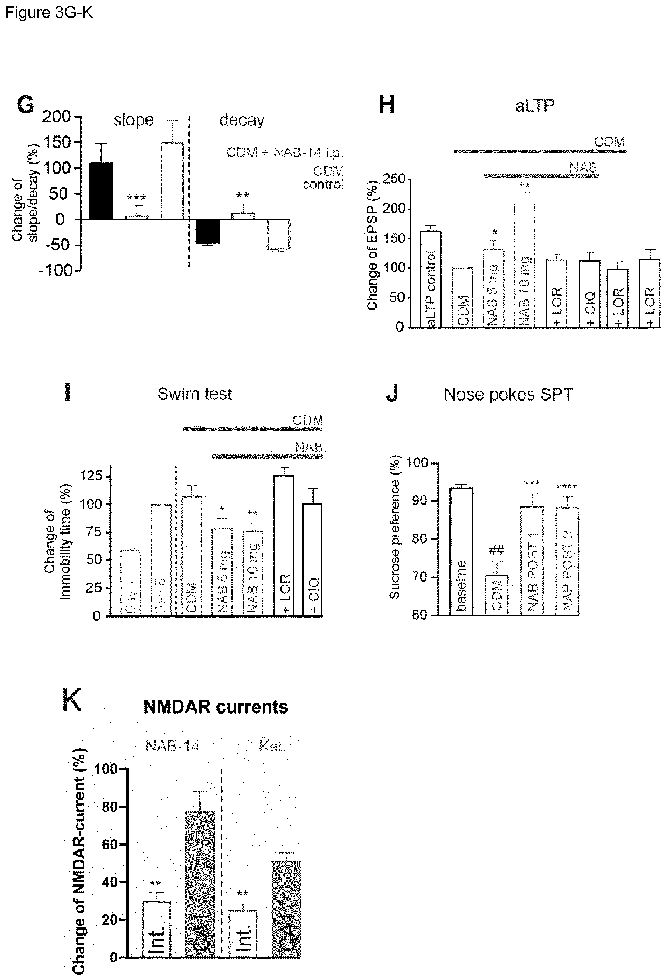

**Figure 3** **- Modulation of GluN2D exerts antidepressive potency**
   **A/B** GluN2C/D antagonist, NAB-14 did not affect basal EPSP-amplitude when added to the bathing solution. **C** NAB-14 was able to enhance liLTP induced increase of EPSP amplitude significantly (compared to control liLTP; Fig. 2G). **D** NAB-14 reversed effects of aLTD application and turned the usually observed decrease of EPSP amplitude (control bar, black) into a significant increase. **E** CDM-induced blockade of LTP was fully reversed by NAB-14 application (25 to 30 min VDM vs. CDM + NAB-14). **F** Overlay of pre LTP protocol and post LTP protocol EPSP-trace. **G** Analysis of graphically determined slope and decay parameters in the control, CDM and CDM + NAB-14 condition. Note that NAB-14 was capable of completely reversing slope increase and decay changes by CDM. H Overview of LTP-experimental series. CDM fully blocked aL TP inducibility, whilst low dose NAB-14 (5µM) partially and high dose NAB (10µM) fully rehabilitated LTP inducibility (CDM vs. NAB 5 µM /NAB10 µM). Co-medication with Lorazepam or GluN2D positive allosteric modulator CIQ blocked NAB-14 effects completely. Lorazepam did block LTP inducibility in control and CDM condition. I Overview of Swim-test analysis. Immobility time increase from day 1 to day 5 and remained stable on the test day (CDM), whilst injection of NAB-14 (5/10µM) significantly reduced immobility time. Comedication with Lorazepam or CIQ blocked NAB-14 effects of swim test analysis. **J** Sucrose preference (measured as nose-pokes-sucrose-preference) was significantly reduced in CDM mice and could be fully reestablished by NAB (5µM) injection during the first (POST1) and the second (POST2) night. **K** Change of pharmacologically isolated (CNQX) NMDAR-currents in CA1 pyramidal cells (CA1) and fluorescent labelled SOM-interneurons (Int.) before and after application of NAB-14 and R,S-ketamine (Ket.). Data are means ±SEM. *P <.05, **P <.01, ***P <.001, ****P <.0001, ##P <.01.

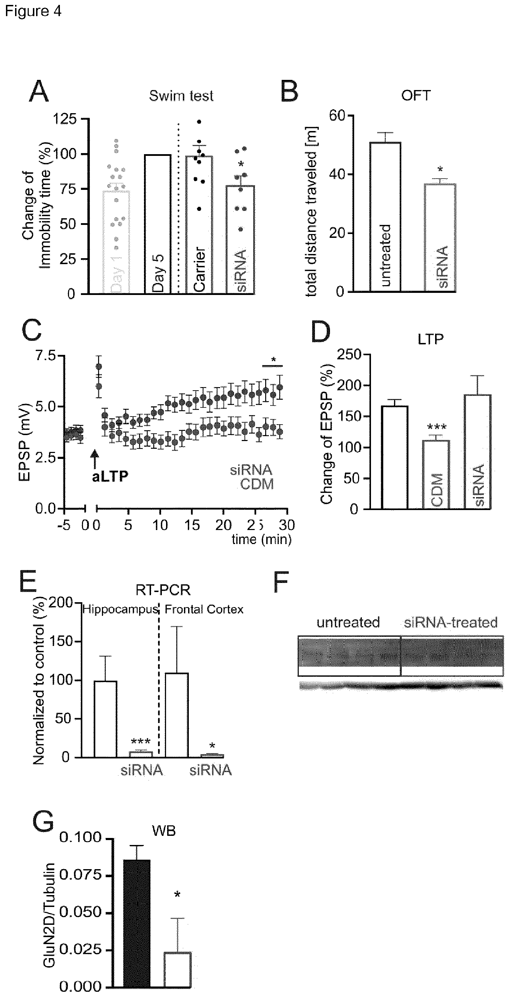

**Figure 4** **- SiRNA approach able to mimic NAB-14 and R,S-ketamine.**
   **A** CDM was applied to mice and resulted in a significant increase of immobility time from day 1 to day 5. Animals receiving carrier substance only injection showed a stable immobility time, whilst animals treated intrathecally with specific siRNA against GluN2D showed a significantly reduced immobility time (carrier substance only vs. siRNA). **B** SiRNA treated animals showed a lower explorative and locomotor activity in the OFT. **C/D** SiRNA injections lead to a reestablishment of stress-induced (CDM) blockade of LTP (min 25 to 30, CDM vs. siRNA). **E** Real-time PCR revealed a significant downregulation of GluN2D-RNA in the hippocampus and the frontal cortex of mice treated with siRNA. F/G Protein downregulation was analyzed by western-blot and showed a significant reduction, normalized on Tubulin. Data are means ±SEM. *P <.05, ***P <.001.

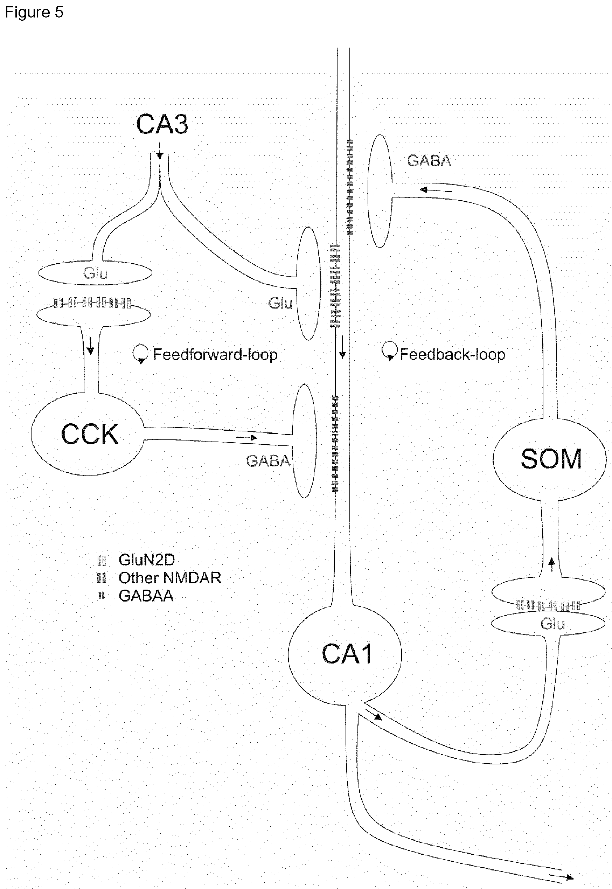

**Figure 5** **- Schema of relevant interneuron network loops for the understanding of disinhibition mechanism in CA1-CA3 synapse of the hippocampus.**
   Stimulation of CA3/Schaffer collaterals leads to excitatory activity at CA3-CA1 synapse and at CA3-CCK synapse. CCK interneurons then release GABA at CCK-CA1 synapse resulting in a hyperpolarization of the dendritic arbor (Feedforward-loop). CA1 dendrite is depolarized by CA3 excitatory activity. CA1 activity stimulates CA1-SOM synapse resulting in a higher activity of SOM interneurons. SOM interneurons then release GABA at SOM-CA1 synapse resulting in a hyperpolarization of the dendritic arbor (Feedback-loop). Receptor composition of excitatory synapses differs substantially, whilst CA3-CA1 synapse mainly consists in NMDAR subunits other than GluN2D, CA3-CCK and CA1-SOM synapses mainly consist in NMDARs containing GluN2D.

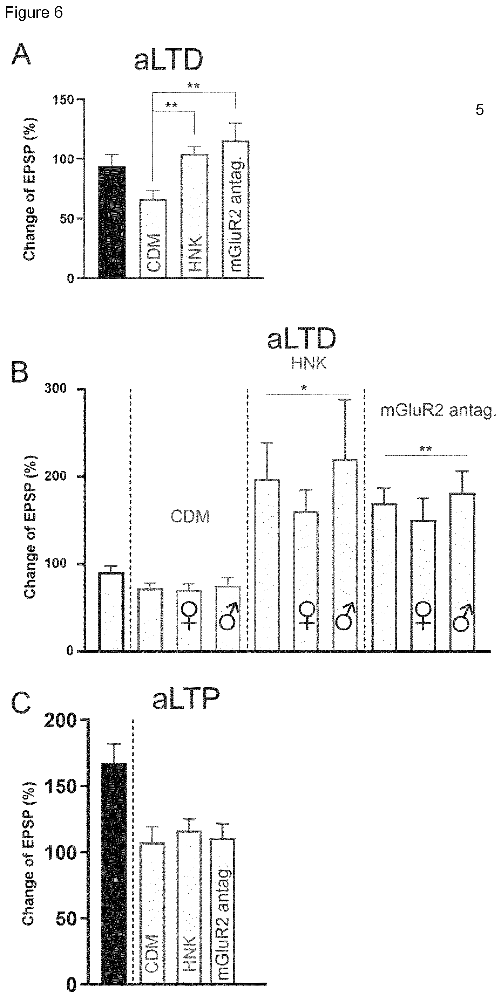

**Figure 6** **- Modulation of mGluR2 rescues stress-induced impairment of synaptic plasticity** .
   **A** ALTD inducibility was facilitated by CDM application. Treatment with HNK or mGluR2 antagonist 1 completely blocked the LTD inducibility in CDM mice, reversing stress-induced changes of plasticity. **B** HNK and mGluR2 antagonist 1 treatment before the application of stress (before CDM) resulted in a complete revers of EPSP amplitudes. Instead of a decrease, EPSP amplitudes significantly increased in male and female mice equally. **C** LTP induction was blocked by CDM application and neither HNK nor mGluR2 antagonist 1 treatment did change this blockade. Data are means ±SEM. *P <.05, **P <.01.

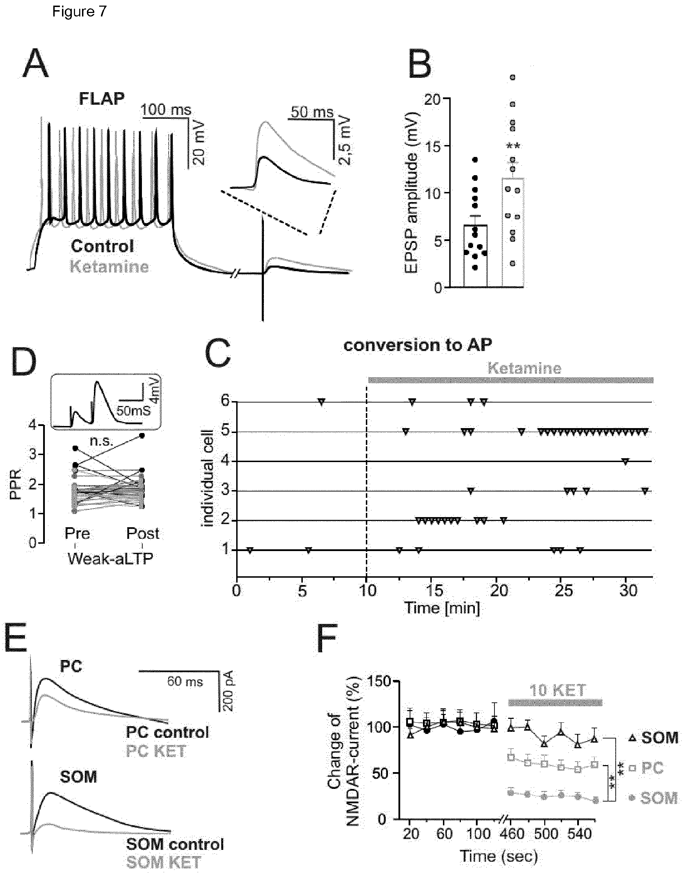

**Figure 7** **- Ketamine preferentially inhibits NMDAR currents in feedback-loop SOM-INs and converts EPSPs in APs.**
   **A** Representative voltage traces from the first 10 min in control solution (black) and at 25 min in the presence of ketamine (10 µM, grey) in the feedback loop-activating protocol (FLAP). Inset: magnified EPSP traces. **B** EPSP amplitudes in the FLAP. After wash-in of 10 µM ketamine (grey), the EPSP amplitudes increased significantly compared to the baseline condition (n = 13). **C** Raster plot demonstrating a significant increase in synaptic AP probability after addition of ketamine to the bathing solution in cells recorded over 30 min (n = 6). After a stable baseline (0-10 min) ketamine (10 µM, grey) was washed in (10-30 min) and Shaffer collateral stimulation resulted more often in an AP compared to baseline. **D** The paired-pulse ratio (PPR, EPSP2/EPSP1) was unchanged before and after weak-aLTP induction in control solution (n = 13) and in the presence of 5 (n = 9; light grey) or 10 (n = 10; dark grey) µM ketamine (inset: representative paired-pulse recording). **E** Representative NMDAR current traces in CA1 PCs and SOMs before (black) and after (grey) application of 10 µM ketamine (KET) to the bath solution. **F** Time course of the maximum amplitude of NMDAR currents (normalized to their initial baseline averages) in PCs (n = 11) and SOMs (n = 9) before and after bath ketamine application or in control solution. Ketamine (10 µM) had a significantly more pronounced inhibitory effect on NMDAR currents in SOMs (grey dots) than in PCs (grey squares). In the control solution, NMDAR current amplitudes in SOM remained stable (black triangles). Data are Means ± SEM. p** < 0.01.

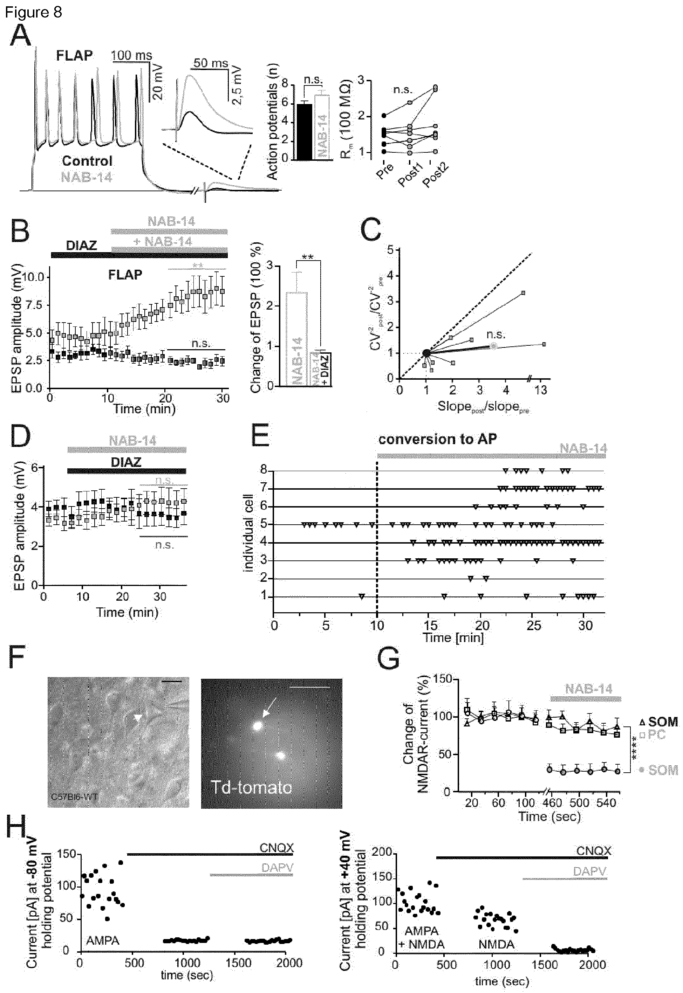

**Figure 8****: NAB-14 specifically inhibits SOM-INs in the feedback loop.**
   **A** Representative voltage traces from the first 10 min in control solution (black) and at 25 min in the presence of NAB-14 (10 µM, grey) in the feedback loop-activating protocol (FLAP). Inset: magnified EPSP traces. Middle panel: there was no significant alteration in AP number under either condition. Right panel: There was no significant change in postsynaptic input resistance (Rm) at min 10, 20 and 30. **B** Time course of maximum EPSP amplitudes in the FLAP. After wash-in of 10 µM NAB-14 (grey), the EPSP amplitudes increased significantly (n = 9). This effect was prevented in the presence of diazepam (3 µM, black/grey, n = 7). **C** CV analysis of the effect of NAB-14 wash-in in the FLAP, consistent with a postsynaptic mechanism. The bolded line connecting dots represents the averages of all analyzed cells (n = 8; lines connecting squares). **D** Time course of maximum EPSP amplitudes after wash-in of 10 µM NAB-14 (grey, n = 9) or 3 µM diazepam (black, n = 7) in the absence of APs. There were no significant pre/post changes. E Raster plot demonstrating a significant increase in synaptic AP probability after addition of NAB-14 to the bathing solution in cells recorded over 30 min (n = 8). After a stable baseline (0-10 min) NAB-14 (10 µM, grey) was washed in (10-30 min) and Shaffer collateral stimulation resulted more often in an AP compared to baseline. **F** Representative differential interference contrast video microscopy (left) and fluorescence (right) image of SOMs (green arrows) in brain slices from wild-type C57BI6 and SOM-Cre (SST tm2.1(cre)Zjh/J) td-Tomato mice (scale bar native image: 20 µm, fluorescent image: 100 µm). **G** Time course of maximum NMDAR current amplitudes in SOM-Ins (n= 9) in control solution (SOM, triangles) and in CA1 PCs (PC, grey squares, n = 11) and SOMs (grey circles, n = 9) after bath application of 10 µM NAB-14. **H** Control experiments indicating that currents recorded at -80 mV (left) could be fully blocked by addition of CNQX (10 µM, black) to the bathing solution, DAPV (100 µM, gray) did not have further effects on the recorded currents. Currents recorded at + 40 mV (right) were only partially influenced by CNQX (10 µM, black) but could be fully blocked by DAPV (100 µM, gray). In summary recordings at +40 mV under the presence of CNQX represent purely NMDAR currents. Data are Means ± SEM. p** < 0.01, p**** < 0.0001
**Figure 9****: QNZ-46 exerts antidepressive effects in CDM.**
   The application of 7 mg/kg QNZ-46 (i.p.) two hours before behavioral readout significantly reduced immobility time in CDM (n = 10). Data are Means ± SEM. p* < 0.05, p*** < 0.001.

### DETAILED DESCRIPTION OF THE INVENTION

As noted above, the inventors have surprisingly found that the GluN2D subunit of the NMDAR or the mGluR2 can be targeted when aiming for the treatment or relapse prevention of a depressive episode. When it comes to GluN2D, this opens up a completely new field since it is now possible to specifically target the subunit of interest vs. a heterogenic population of NMDARs, which was thus far the target of R,S,-Ketamine. In order to arrive at the above conclusion and as shown in the examples of the present application, treatment with R,S-ketamine, S-ketamine, NAB-14 and a GluN2D siRNA reduced depression symptoms in a well-known and well-accepted model system of depression, namely the chronic despair model mice (CDM; Serchov et al.; Holz et al.; Vestring et al.), in terms of a reduced immobility time, a renormalized sucrose preference, and restoration of altered long-term potentiation and long-term depression in hippocampal brain slices. Accordingly, two structurally very different GluN2D inhibitors were successfully tested, namely small molecule inhibitors and a nucleic-acid based siRNA. It is noteworthy that the two small molecules that were tested, NAB-14 and QNZ46, are not related in terms of their scaffold but have completely different chemical structures.

It is important to understand that GluN2D-inhibitors have been tested previously, but not in a setup that would allow for a conclusion on their efficacy in treating depression, let alone a well-known and well-accepted model system for depression. First of all, it should be mentioned that GluN2D-knock-out systems (which are different from knock-down systems, where GluN2D was present and functional before the knock-down) including in particular GluN2D-knock-out mice do generally not allow for any conclusion on depression, as GluN2D was never expressed in the system (in particular not in the knock-out mice) and was thus never present to begin with. It is therefore simply not possible to draw a conclusion as regards the treatment of depression because there is no well-known and well-accepted model system for depression in a GluN2D-knock out system such as, in particular, GluN2D-knock out mice. Second, publications concerned with GluN2D-inhibitors must be analyzed carefully with respect to the actual data gained therein, with the result that e.g. Zhang et al., which is concerned with the GluN2D-inhibitor QNZ46, fails to actually test QNZ46 for an antidepressive effect or that Ide et al. completely fails to test any GluN2D-inhibitors. In other words, there are no experimental data in the prior art that show that a GluN2D-inhibitor would be ineffective in a treatment model for depression. As a result, the experimental data gained by the present inventors are not in contradiction to data gained previously, e.g. data gained in Zhang et al. and Ide et al. Rather, the present inventors were simply the first to test the GluN2D-inhibitors in a well-known and well-accepted model system of depression.

Considering the hypothesis postulated by Zhang et al. and Ide et al. based on the data gained by them, the present inventors were not only the first to test the GluN2D-inhibitors, including QNZ46 (for which data has also been gained by Thang et al., see above), for their efficacy in depression treatment but they carried out these experiments against the hypothesis postulated by Zhang et al. and Ide et al., who basically postulate that GluN2D agonists (and not antagonists) would be required to treat depression, such that GluN2D agonists could optionally be used in combination with ketamine. Example 7 demonstrates that, contrary to the hypothesis of Zhang et al. and Ide et al., QNZ46 exerts antidepressive effects just like the other GluN2D inhibitors tested herein. The inventors therefore experimentally demonstrate that two structurally unrelated small molecule GluN2 inhibitors and a nucleic acid based siRNA all exert antidepressive effects in a manner that goes against the hypothesis posited by Zhang et al. and Ide et al.

It is desirable for both, the GluN2D inhibitor and the mGluR2 inhibitor of the present invention, that they inhibit the target as selective as possible. The more selective the inhibition, the fewer side effects are expected to occur.

The terms "selective", "selectively" and "selectivity" are used herein for the inhibition of GluN2D in the meaning that the respective inhibitor is more selective for the GluN2D subunit compared to a GluN2A /GluN2B subunit of the NMDAR. Such a compound is e.g. NAB-14 as tested herein, which is < 800-fold selective for recombinant GluN2D (and also GluN2C) over GluN2A/GluN2B in Xenopus oocytes and has an IC50 value of 580 nM at recombinant GluN2D-containting receptors expressed in mammalian cells (Swanger et al). Such a compound is e.g. a siRNA, which is sequence specific for the sequence of GluN2D or its upstream regulatory region, and will therefore result exclusively in the downregulation of the GluN2D-subunit but not e.g. the GluN2C-subunit.

The terms "selective", "selectively" and "selectivity" are used herein for the inhibition of mGluR2 in the meaning that the respective inhibitor is more selective for the mGluR2 compared to a different receptor, in particular different G-protein coupled receptors, more particularly mGluR1 (glutamate receptor 1), mGluR3 (glutamate receptor 3), mGluR4 (glutamate receptor 4), mGluR6 (glutamate receptor 6), mGluR7 (glutamate receptor 7), mGluR8 (glutamate receptor 8). Such a compound is e.g. mGluR2 antagonist 1 as tested herein, which has an IC50 value of 8.9 nM as measured by FLIPR assay in CHOdhfr-cells expressing human mGluR2 compared to IC50s of >10000 nM for mGluR1, mGluR3, mGluR4, mGluR7, and mGluR8 and of 9220 nM for mGluR6 (see Shu et al.).

In view of the above, the present application is in particular directed to GluN2D inhibitors that selectively inhibit GluN2D, wherein the selectivity is at least a selectivity that is higher when compared to GluN2A/GluN2B inhibition. In other words, the GluN2D inhibitor of the present invention is more selective for GluN2D compared to GluN2A or GluN2B. Further, the present application is in particular directed to mGluR2 inhibitors that selectively inhibit mGluR2, wherein the selectivity is at least a selectivity that is higher when compared to other G-protein coupled receptors, in particular mGluR1, mGluR3, mGluR4, mGluR6, mGluR7, or mGluR8. In other words, the mGluR2 inhibitor of the present invention is more selective for mGluR2 compared to a different G-protein coupled receptor, in particular mGluR1, mGluR3, mGluR4, mGluR6, mGluR7, or mGluR8.

It is obvious that the selectivity depends on the type of inhibitor that is used to inhibit GluN2D and mGluR2, respectively.

If a nucleotide-based inhibitor is used, in particular an antisense oligonucleotide, a siRNA, a shRNA, a miRNA or a sgRNA, this inhibitor is selective already as a result from its design, namely in that such an inhibitor is sequence-specific for the sequence of GluN2D or mGluR2, respectively.

The same applies to an antibody directed to GluN2D or mGluR2, respectively, since the antibody is raised and designed such that it binds selectively to GluN2D or mGluR2, respectively.

When it comes to a small molecule targeting GluN2D or mGluR2, respectively, the present application provides data that such small molecules are effective (see example 3 for NAB-14 and example 4 for mGluR2 antagonist 1). Moreover, these data are also relevant for a small molecule that selectively targets GluN2D or mGluR2, respectively, in the meaning of the present invention: both NAB-14 and mGluR2 antagonist 1 have been shown to be selective for their targets (see Swanger et al; Shu et al). Accordingly, the data of the present application support that a targeting as well as the preferred selective targeting of GluN2D or mGluR2, respectively, by small molecules is effective. Further such preferred selective small molecule inhibitors may be screened for by the skilled person in accordance with the binding experiments and selectivity experiments as carried out for a GluN2D small molecule inhibitor in Swanger et al. and for a mGluR2 small molecule inhibitor in Shu et al. Thus, examples for (selective) small molecule inhibitors for both targets (NAB-14 for GluN2D and mGluR2 antagonist 1 for mGluR2) are disclosed and supported by data herein, and assays to identify further inhibitors with (slightly) different structures are disclosed in the afore-mentioned prior art such that the skilled person can easily identify such further inhibitors.

In more detail, small molecular inhibitors that fulfill the selectivity-requirement as outlined above may be identified by determining their selectivity by IC₅₀ measurements for the GluN2D subunit of the NMDAR. Such a method for determining IC₅₀ for each of the GluN2 subunits of the NMDAR, namely two-electrode voltage-clamp recordings in Xenopus oocytes, is e.g. described in Swanger et al. Briefly, Xenopus laevis oocytes are transfected with cRNAs for GluN1 and the GluN2 subunit of choice according to standard procedures, and concentration-response curves for compounds to be tested as inhibitors are generated by applying a maximally effective concentration of glutamate (100 µM) and glycine (30 µM), followed by variable concentrations of test compound up to 100 µM. 2-hydroxypropyl-fl-cyclodextrin (1-10 mM) is added to the recording solution for Xenopus oocyte recordings to ensure that the compounds remain in solution. Concentration-response data is analyzed using OriginPro 9.0 or GraphPad 5.0. For inhibition concentration-response curves, the inhibitory response evoked by test compounds is given as a percentage of the initial response to glutamate and glycine alone. Data for individual cells is fit with the Hill equation: $response = \left(100-minimum\right) / \left(1+{\left(\left[I\right]/{IC}_{50}\right)}^{N}\right) + minimum$ where N is the Hill slope, [I] is the inhibitor concentration, and minimum is the minimum response predicted for saturating concentrations of inhibitor. Minimum is fixed to 0 unless stated otherwise. For graphical representation, the data are normalized to the maximum response, averaged across all cells, and fit with the Hill equation. As noted above, an exemplary selective small molecule inhibitor is NAB-14 as tested herein, which is < 800-fold selective for recombinant GluN2D (and also GluN2C) over GluN2A/GluN2B in Xenopus oocytes and has an IC50 value of 580 nM at recombinant GluN2D-containting receptors expressed in mammalian cells (Swanger et al).

Before the present invention is described in more detail in the example section, the following definitions are introduced.

### 1. Definitions

As used in the specification and the claims, the singular forms of "a" and "an" also include the corresponding plurals unless the context clearly dictates otherwise.

The term "about" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising". If hereinafter a group is defined to comprise at least a certain number of embodiments, this is also meant to encompass a group which preferably consists of these embodiments only.

The term "GluN2D" as used herein refers to a specific GluN2 subunit of N-methyl-D-aspartate receptor (NMDAR), namely the subunit GluN2D (and thus a protein). GluN2D is almost exclusively expressed on inhibiting interneurons. The underlying gene in Genbank is the gene with the Gene ID: 2906 (updated on 11-Jun-2021), with the official Symbol GRIN2D, which is also known as GluN2D, EB11, NR2D, DEE46, EIEE46 and NMDAR2D.

NMDARs are inotropic glutamate receptors that mediate excitatory neurotransmission in the central nervous system. The NMDARs are heterotetrameric complexes composed of two obligatory GluN1 subunits and generally two GluN2 subunits. There are four types of GluN2 subunits, namely GluN2A, GluN2B, GluN2C and GluN2D. The various GluN2 subunits have unique developmental and cell-type specific expression patterns. Subunit composition determines the electrophysiological and pharmacological properties of NMDARs. The GluN2C- and GluN2D-containing receptors have lower sensitivity to Mg²⁺-block compared to GluN2A- and GluN2B-containing receptors, lack desensitization and have high affinity for glutamate and glycine. Although similar in several aspects, GluN2C- and GluN2D-containing receptors also diverge in several biophysical and pharmacological properties. For example, in the presence of Mg²⁺, GluN1/GluN2C receptors exhibit higher blockade with R,S-ketamine compared to GluN1/GluN20 receptors or other NMDAR subtypes (see Khlestova et al.).

The term "GluN2D inhibitor" as used herein refers to any compound capable of interfering with GluN2D's activity and/or assembly into a functional NMDAR and/or presence in a cell. Thus, the term includes compounds capable of reducing or preventing the transcription and/or translation of the GluN2D gene (which encodes GluN2D) and/or the stability of the GluN2D mRNA or GluN2D or reducing or preventing its interaction with other molecules, e.g. other NMDAR subunits. Reduction can mean e.g. at least 50 %, preferably at least 80 %, more preferably at least 90 %, even more preferred by at least 95 % reduction compared to the situation in the absence of the inhibitor. For inhibitors that interact directly with GluN2D, activity reduction can be tested as described in Example 3. For inhibitors that affect transcription, GluN2D transcript levels after treatment with the inhibitor can be compared to GluN2D transcript levels in the absence of the inhibitor to determine reduction by state of the art methods. For inhibitors that affect translation, GluN2D protein levels after treatment with the inhibitor can be compared to GluN2D protein levels in the absence of the inhibitor by state of the art methods. The present application successfully tested two different GluN2D-inhibitors in a well-accepted animal model for depression, namely a small molecule inhibitor (NAB-14) and a siRNA. These two inhibitors have a completely different mechanism of action but share the function of GluN2D-inhibition, which results in the treatment of depression, as shown by the examples of the present application. Accordingly, these data show that it is not crucial how the GluN2D-inhibition is achieved - what matters is that GluN2D is inhibited, with the result that depression is treated. In other words, a concept fit for generalization is provided in the present application.

The term "mGluR2" as used herein refers to G-protein coupled metabotropic glutamate receptor 2. mGluR2 is expressed extrasynaptically, predominantly on presynaptic neurons, and has been shown to normalize excessive glutamate levels and increased synaptic activity of glutamate in this region. mGluR2 functionally acts as an inhibiting autoreceptor. The underlying gene in Genbank is the gene with the Gene ID: 443084 (updated on 12-Oct-2019), with the official Symbol MGLUR2.

The term "mGluR2 inhibitor" as used herein refers to any compound capable of interfering with mGluR2's activity and/or presence in a cell. Thus, the term includes compounds capable of reducing or preventing the transcription and/or translation of the mGluR2 gene (which encodes mGluR2) and/or the stability of the mGluR2 mRNA or mGluR2 or reducing or preventing its interaction with other molecules. Reduction can mean e.g. at least 50 %, preferably at least 80 %, more preferably at least 90 %, even more preferred by at least 95 % reduction compared to the situation in the absence of the inhibitor.

The term "treatment or relapse prevention" as used herein refers to any type of a beneficial effect, e.g. amelioration of at least one symptom of a disease or disorder. A beneficial effect can take the form of an improvement over baseline, e.g. with regard to severity of a depressive episode or frequency of depressive episodes. An effective treatment may e.g. reduce frequency of depressive episodes, reduce the intensity of a depressive episode, or prevent a depressive episode. The current treatment standard is to treat a patient for about six months starting at an initial depressive episode, after which about six months the treatment is ceased if there are no factors putting the patient at increased risk for future depressive episodes, i.e. relapse. If the patient however has experienced multiple episodes of depression or is currently experiencing high stress conditions, then relapse prevention may be considered, i.e. treatment may be extended beyond six months from the initial depressive episode. That is, relapse prevention begins after the initial treatment period of six months from the first depressive episode. Standard treatment modalities are outlined in the respective national treatment guidelines, e.g. at https://www.awmf.org/leitlinien/aktuelle-leitlinien.html.

The term "depressive episode" as used herein refers to mild, moderate, or severe depressive episodes, preferably depressive episodes occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34. When reference is made herein to ICD-10, this refers to "ICD-10-GM Version 2021, Systematisches Verzeichnis, Internationale statistische Klassi-fikation der Krankhei-ten und verwandter Gesundheitsprobleme, 10. Revision, Stand: 18. September 2020" published by Bundesinstitut für Arzneimittel und Medizinprodukte (BfArM) im Auftrag des Bundes-ministe-riums für Gesundheit (BMG) unter Beteiligung der Arbeitsgruppe ICD des Kuratoriums für Fra-gen der Klassifikation im Gesundheitswesen (KKG) in Cologne, Germany, in 2020, file name "icd10gm2021_syst_odt_20200918.pdf" available at https://www.bfarm.de/SharedDocs/Downloads/DE/Kodiersysteme/klassifikationen/icd-10-gm/vorga-enger/icd10gm2021_zip.html?nn=841246&cms_dlConfirm=true&cms_calledFromDoc=841246. In particular, a patient experiencing a depressive episode suffers from lowering of mood, reduction of energy, and decrease in activity. Capacity for enjoyment, interest, and concentration is reduced, and marked tiredness after even minimum effort is common. Sleep is usually disturbed and appetite diminished. Self-esteem and self-confidence are almost always reduced and, even in the mild form, some ideas of guilt or worthlessness are often present. The lowered mood varies little from day to day, is unresponsive to circumstances and may be accompanied by so-called "somatic" symptoms, such as loss of interest and pleasurable feelings, waking in the morning several hours before the usual time, depression worst in the morning, marked psychomotor retardation, agitation, loss of appetite, weight loss, and loss of libido.

Depending upon the number and severity of the symptoms, a depressive episode may be specified as mild, moderate or severe. In mild depressive episodes, two or three of the above symptoms are usually present. The patient is usually distressed by these but will probably be able to continue with most activities. In moderate depressive episodes, four or more of the above symptoms are usually present and the patient is likely to have great difficulty in continuing with ordinary activities. In severe depressive episodes without psychotic symptoms, several of the above symptoms are marked and distressing, typically loss of self-esteem and ideas of worthlessness or guilt. Suicidal thoughts and acts are common and a number of "somatic" symptoms are usually present. In severe depressive episodes with psychotic symptoms, differs thereto by the presence of hallucinations, delusions, psychomotor retardation, or stupor so severe that ordinary social activities are impossible; there may be danger to life from suicide, dehydration, or starvation. The hallucinations and delusions may or may not be mood-congruent. Other depressive episodes which can occur as part of a disorder as defined by ICD-10: F31-34 are further listed in ICD-10: F31-34.

Depressive episodes may occur in isolation, i.e. constitute the entirety of a disorder, (as defined in ICD-10: F32) or as part of other disorders as defined in ICD-10: F31 (bipolar affective disorder), F33 (recurrent depressive disorder), and F34 (persistent mood [affective] disorder). Depressive episodes might also occur in bipolar affective disorder (ICD-10: F31). Bipolar affective disorder is a disorder characterized by two or more episodes in which the patient's mood and activity levels are significantly disturbed, this disturbance consisting on some occasions of an elevation of mood and increased energy and activity (hypomania or mania) and on others of a lowering of mood and decreased energy and activity (depression). Repeated episodes of hypomania or mania only are classified as ICD-10 F30.

As used herein, depressive episodes do not refer to symptoms that occur as part of a disorder as defined by ICD-10: F06. In disorders as defined by ICD-10: F06, as opposed to ICD-10: F31-F34 (defining mood affective disorders), the cause of such symptoms lies in brain damage and dysfunction and/or physical disease. Examples of such brain damage and dysfunction or physical disease include white matter lesions, demyelination, and myelin injury.

The Diagnostic and Statistical Manual of Mental Disorders (DSM-5) is published by the American Psychiatric Association and is the predominant psychiatric classification system in the US. There are some differences between DSM-5 and ICD-10. DSM-5 uses the term "major depressive disorder (MDD)", which is encompassed by the term "depressive episode". The diagnosis hinges on the presence of a single or recurrent major depressive episode. Further qualifiers are used to classify both the episode itself and the course of the disorder. The category depressive disorder not otherwise specified is diagnosed if the depressive episode's manifestation does not meet the criteria for a major depressive episode. The ICD-10 system does not use the term major depressive disorder, but lists very similar criteria for the diagnosis of a depressive episode (mild, moderate, or severe); the term recurrent may be added if there have been multiple episodes without mania.

DSM-5 recognizes further subtypes of MDD, called specifiers, in addition to noting the length, severity, and presence of psychotic features, wherein also these subtypes of MDD are encompassed by the term MDD and depressive episode, respectively:
With anxious distress
With mixed features
With melancholic features
With atypical features
With mood-congruent psychotic features
With mood-incongruent psychotic features
With catatonia
With peripartum onset
With seasonal pattern (recurrent episode only)

Moreover, DSM-5 has introduced the term "Persistent Depressive Disorder (Dysthymia)", which represents a long-lasting (>2 years) but less severe depressive condition. All of these subtypes are by consequence also encompassed by the term "depressive episode".

MDD is one of the most prevalent forms of mental illness and causes an enormous individual suffering and socioeconomic damage, affecting more than 10% of the population during their lifetime. Despite its importance, current treatment options are limited, and conventional antidepressants take weeks or even months to reduce the patients' symptoms. This delay of onset has been associated with an increase of suicidal ideation; which, together with a high rate of patients (> 30%) that do not respond even after multiple treatment attempts, point out the urgent need for novel and rapid acting antidepressants.

The term "depression" as present in the term "depressive episode" can be defined as follows:
First, depression can be divided into several types. Major depression is the most severe form of depression characterized by a severe, persistent (greater than 2 weeks) depressed mood and loss of interest or pleasure in normal activities accompanied by decreased energy, changes in sleep habits, restless behavior, difficulty concentrating, loss of appetite, feelings of guilt or hopelessness, and, in severe cases, psychotic symptoms such as hallucinations, delusions, and even suicidal thoughts. The Beck's Depression Scale Inventory, or other screen tests for depression. can be helpful in diagnosing depression.

A second form of depression is chronic low-grade depression, also known as dysthymia. Dysthymia is present most of the time for a period of two or more years wherein an individual experiences a decrease in his/her overall level of energy, appetite, and sleep, as well as has feelings of low self-esteem and hopelessness. These symptoms cause distress and the individual has difficulty functioning in everyday activities. These symptoms, however, are not as severe as those symptoms experienced in major depression The cause and maintenance of these symptoms are typically due to one of the following problems: loss of a friend, substantial disappointment at work or home, prolonged or chronic illness, and alcohol or drug abuse. People who suffer from dysthymia are at an increased risk for episodes of major depression. This produces a behavioral pattern called "double depression" wherein the individual is mildly depressed most of the time, with periodic symptoms of major depression.

The least severe form of depression is a depressed mood. This is an emotional state dominated by feelings of sadness, gloominess, or emptiness, which may be associated with lack of energy. Depressed moods are usually temporary responses to an unhappy or stressful event.

As opposed to unipolar major depression. the incidence of bipolar disorder does not vary widely around the world. The exact cause is unknown, but it is linked to areas of the brain which regulate mood, and has a strong genetic component. The American Psychiatric Association's "Diagnostic and Statistical Manual of Mental Disorders" describes two types of bipolar disorder, type I and type II. In the type I (formerly known as manic depressive disorder), there has been at least one full manic episode. People with this type, however, may also experience episodes of major depression. In type II disorder, periods of "hypomania" involve more attenuate (less severe) manic symptoms that alternate with at least one major depressive episode. When the patients have an acute exacerbation, they may be in a manic state, depressed state, or mixed state. The manic phase is characterized by elevated mood, hyperactivity, over-involvement in activities, inflated self-esteem, a tendency to be easily distracted, or little need for sleep. In the depressive phase, there is loss of self-esteem, withdrawal, sadness, or a risk of suicide. Either the manic or the depressive episodes can predominate and produce a few mood swings, or the patterns of the mood swing may be cyclic. While in either phase, patients may abuse alcohol or other substances, which worsens the symptoms.

In an embodiment of the present invention, treatment or relapse prevention of a depressive episode includes treatment or relapse prevention of bipolar affective disorder and MDD.

"Dissociation" as used herein means a pathological phenomenon in which a person feels disconnected from their body.

The term "disinhibition" as used herein refers to any mechanism by which inhibition of neuronal cells at a synapse is prevented or reversed. Disinhibition may be direct or indirect. GluN2D-mediated activity is an example of indirect disinhibition, as GluN2D is almost exclusively expressed on inhibiting interneurons (see also SOM in Figure 5). These inhibitory interneurons, when activated by postsynaptic neurons via GluN2D-containing NMDAR, then inhibit these same postsynaptic neurons by hyperpolarization of their dendritic arbor via GABA (see also CA1 in Figure 5). This feedback loop is depicted in Figure 5. Inhibition of the inhibitory interneurons by inhibiting GluN2D therefore blocks inhibition of postsynaptic neurons via these inhibitory interneurons, i.e. breaks the feedback loop depicted in Figure 5. On the other hand, mGluR2-mediated activity is an example of direct disinhibition, as mGluR2 is predominantly expressed on presynaptic neurons and functionally acts as an inhibiting autoreceptor. Accordingly, activation of mGluR2 leads to inhibition of presynaptic neurons. Inhibition of mGluR2 activation therefore prevents (auto-)inhibition of the presynaptic neuron, i.e. breaks the autoregulatory inhibition loop in the presynaptic neuron.

The term "synaptic plasticity" as used herein refers to the change that occurs at synapses, the junctions between neurons that allow them to communicate. Synaptic plasticity controls how effectively two neurons communicate with each other. The synaptic strength is not static, but rather can change in both the short term and long term. Synaptic plasticity refers to these changes in synaptic strength. Short-term synaptic plasticity refers to changes in synaptic strength that occur on a sub-second timescale: a rapid up or down adjustment of the volume control that helps determine how important that connection is to the ongoing conversation, but which reverts to "normal" soon afterwards. Long-term synaptic plasticity lasts anywhere from minutes to hours, days, or years. Long-term plasticity is the dominant model for how the brain stores information. "Increasing synaptic plasticity" means that greater variation in synaptic strength is enabled. Synaptic strength is increased, e.g. by inhibition of inhibitory interneurons, or by inhibiting autoinhibition of presynaptic neurons.

The term "small molecule" as used herein refers to a small organic compound having a low molecular weight. A small molecule may be a synthetic compound not known to occur in nature or a naturally-occurring compound isolated from or known to occur in natural sources, such as e.g. cells, plants, fungi, animals and the like. A small molecule in the context of the present invention preferably has a molecular weight of less than 5000 Dalton, more preferably of less than 4000 Dalton, more preferably less than 3000 Dalton, more preferably less than 2000 Dalton or even more preferably less than 1000 Dalton. In a particularly preferred embodiment a small molecule in the context of the present invention has a molecular weight of less than 800 Dalton. In another preferred embodiment, a small molecule in the context of the present invention has a molecular weight of 50 to 3000 Dalton, preferably of 100 to 2000 Dalton, more preferably of 100 to 1500 Dalton and even more preferably of 100 to 1000 Dalton. Most preferably, a small molecule in the context of the present invention has a molecular weight of 100 to 800 Dalton. It can be preferred that a small molecule in the context of the present invention meets the "Rule of Five" as set out below and is thus orally active (i.e. has a good oral bioavailability). These rules are as follows: (i) the small molecule has no more than five hydrogen bond donors (e.g. nitrogen or oxygen atoms with one or more hydrogen atoms); (ii) the small molecule has not more than ten hydrogen bond acceptors (e.g. nitrogen or oxygen atoms); (iii) the small molecule has a molecular mass of less than 500 Dalton; (iv) the small molecule has an octanol-water partition coefficient log P not greater than 5.

Exemplary small molecule inhibitors of GluN2D include N-aryl benzamide-based compounds (e.g. NAB-14) (Swanger at al.), dihydroquinoline-pyrazoline (DQP)-based compounds (US 2021/0017149 A1 and US 2016/0368897 A1), quinazoline-4-one derivatives (e.g. QNZ-46) (Mosley et al.), (±)-cis-1-(phenanthren-2yl-carbonyl)-piperazine-2,3-dicarboxylic acid (PPDA) and analogues thereof such as [(2R*,3S*)-1-(phenanthrene-3-carbonyl)piperazine-2,3-dicarboxylic acid (UBP141) and (2R*,3S*)-1-(9-bromophenanthrene-3-carbonyl)piperazine-2,3-dicarboxylic acid (UBP145) (Costa et al.).

The term "antibody" as used herein refers to polyclonal or monoclonal antibodies which specifically bind to an antigen, i.e. GluN2D or mGluR2. The antibody may be an IgG, IgM, IgD, IgA or IgE antibody, with IgG being preferred. "Antigen-binding fragments", i.e. fragments of a whole antibody that maintain their ability to bind the antigen for which the antibody is specific, include Fab, F(ab)2 or scFv fragments. The antibodies or antigen-binding fragments may be conjugated to produce derivatives. Derivatives may include glycosylation variants or obtained by cross-linking to produce aggregates.

Antibodies may be generated by means known in the art. For example, antibodies can be generated by immunizing laboratory animals. The B cells producing the relevant antibodies can be fused with myeloma cells to produce hybridoma cells which can be taken into culture for the production of the antibodies. Methods for purifying the antibodies from the medium are known. For example, Protein A, Protein G or Protein A/G, Ion exchange Chromatography (IEX) or Hydrophobic interaction chromatography (HIC) are known to the person skilled in the art.

"Antisense oligonucleotides" act by hybridizing to target mRNA, in the present case GluN2D or mGluR2 mRNA. Depending on backbone modifications of the oligonucleotide, degradation may occur due to RNase H. The design of suitable antisense oligonucleotides for given target sequences is known to the person skilled in the art (see e.g. Aarstma-Rus et al.). Preferably, the antisense oligonucleotide is capable of binding to and/or is at least partially complementary to a region of the GluN2D or mGluR2 gene or regulatory elements in its close vicinity.

"Small interfering RNAs (siRNAs)" lead to a transient sequence-specific gene silencing. The design of suitable sequences and even software for the design of suitable sequences is available to the skilled person (see e.g. Naito & Ui-Tei). Preferably, the siRNA is capable of interfering with the gene expression of the GluN2D or mGluR2 gene and comprises a first strand at least partially complementary to 15 nucleotides of the GluN2D or mGlur2 gene, and a second strand of 15 to 30 nucleotides in length, wherein at least 12 nucleotides of the first strand and second strand are complementary to each other and form an siRNA duplex. siRNAs include RNA-DNA hybrids in which an siRNA duplex has an asymmetric DNA overhang. Examples thereof are the siRNA formed by the strands with the sequences of SEQ ID NO: 1 and SEQ ID NO: 2 and the siRNA formed by the strands with the sequences of SEQ ID NO: 3 and SEQ ID NO: 4.

"Short hairpin RNAs (shRNAs)" allow for high potency and sustainable effects while an siRNA effect is of transient nature. Design tools for shRNA targeting the GluN2D mRNA or mGluR2 mRNA are also available to the person skilled in the art. After delivery of the shRNA expression vector into the cytoplasm, the vector is transported into the nucleus for transcription (see Rao et al. for a review).

A "microRNA (miRNA)" is a small non-coding RNA molecule that functions in RNA silencing and post-transcriptional regulation of gene expression. miRNA resemble the siRNAs of the RNAi pathway. Design tools for miRNAs targeting the GluN2D mRNA or mGluR2 mRNA are available to the person skilled in the art (see Chen et al. for a review).

A "CRISPR-guide RNA (sgRNA)" functions in CRISPR-based genome editing that requires two components: the guide RNA and a CRISPR-associated endonuclease protein (Cas) or a derivative or fusion thereof. The guide RNA directs the Cas nuclease to the specific target DNA sequence, i.e. the GluN2D gene (also known as GRIN2D gene) or its upstream regulatory elements, via (at least partial) complementarity between the target DNA sequence and part of the guide RNA, and the Cas nuclease then cuts the DNA at that site resulting in a double-strand break. The cell tries to repair it e.g. via non-homologous end to end joining, which is prone to errors by the insertion or deletion of bases which can lead to protein disruption and is the preferred pathway for knocking out a particular gene. In a further preferred embodiment, the gene expression is inhibited by CRISPR interference (CRISPRi). CRISPRi uses the sequence-specific binding of a Cas9/sgRNA complex to the gene. Since instead of the active Cas9, a variant thereof designated dCas9 is used, which carries specific mutations to inactivate the endonuclease function, the dCas9/sgRNA complex does not cleave DNA strands. Due to the binding of the complex to the DNA strand, gene transcription is inhibited by blocking of RNA polymerases. In a particularly preferred embodiment the dCas9 may further comprise a protein domain of e.g. Krüppel associated box (KRAB), whereby the transcription of the bound gene in human cells is reduced up to 50 %, preferably up to 80 %, more preferably up to 90 %, particularly preferred up to 99 %. Design tools for the guideRNA suitable for directing a Cas nuclease to the GluN2D or mGluR2 gene are available to the person skilled in the art; see e.g. from the website of the Broad Institute (https://portals.broadinstitute.org/gpp/public/analysis-tools/sgrna-design).

The term "at least partially complementary" as used herein means at least 50%, at least 60%, at least 70%, at least 80%, at least 85%, at least 86%, at least 87%, at least 89%, at least 90%, at least 91%, at least 92%, at least 93%, at least 94%, at least 95%, at least 96%, at least 97%, at least 98%, at least 99%, or 100% complementary. If there is 100% complementary, the sequence is not partially complementary but fully complementary, which is commonly referred to simply as "complementary". "Partially complementary" can also mean less than 10, less than 9, less than 8, less than 7, less than 6, less than 5, less than 4, less than 3, less than 2, or only 1, but at least 1, base mismatch(es).

The term "vector" as used herein refers to a circular or linear, single-stranded or double-stranded nucleic acid, in particular DNA or RNA. Such a vector typically comprises further genetic information encoding e.g. proteins, such as e.g. viral proteins that are necessary for the transduction of a host cell. If a simple DNA vector is referred to, this is typically a plasmid that comprises in particular certain markers for selection and /or detection, and optionally an origin of replication.

As used herein the term "hybridize" or "hybridizes" refers to the hybridization of a first to a second polynucleotide, which is a process where complementary sequences between the first and the second polynucleotide anneal (thus arriving at an "at least partially complementary" sequences e.g. in the first polynucleotide). To determine if two polynucleotides hybridize to each other, the skilled person will preferably conduct hybridization experiments *in vitro* under moderate or stringent hybridization conditions. Hybridization assays and conditions are known to those skilled in the art and can be found, for example, in Current Protocols in Molecular Biology, John Wiley & Sons, N. Y., 6.3.1-6.3.6, 1991. Stringent conditions may e.g. be conditions in which hybridization takes place in 6X sodium chloride/sodium citrate (SSC) at 45°C, followed by a wash in 0.2 X SSC, 0.1 % SDS at 65°C.

### 2. Pharmaceutical compositions comprising small molecules and antibodies

The inhibitor of the present invention may be a small molecule targeting GluN2D or mGluR2, or an antibody directed to GluN2D or mGluR2, or an antigen-binding fragment thereof, and this inhibitor is a pharmaceutically active agent.

A "pharmaceutically active agent" as used herein means that the respective agent is potent of modulating a response in a patient, i.e. a human or animal being *in vivo.* The term "pharmaceutically acceptable excipient" as used herein refers to excipients commonly comprised in pharmaceutical compositions, which are known to the skilled person. Such excipients are exemplary listed below. In view of the definition "pharmaceutically active agents" as given above, a pharmaceutically acceptable excipient can be defined as being pharmaceutically inactive.

The pharmaceutical compositions featured herein are administered in a dosage sufficient to inhibit GluN2D or mGluR2. In general, a suitable dose of a small molecule or an antibody may be in the range from 1 µg to 100 mg, preferably in the range from 10 µg to 10 mg per day.

A pharmaceutical composition comprising an inhibitor for use according to the present invention may be formulated for oral, buccal, nasal, rectal, topical, transdermal or parenteral application. Oral application can be preferred. Parenteral application can also be preferred and includes intravenous, intramuscular or subcutaneous administration. A dosage form of the present invention may also be designated as formulation or pharmaceutical composition.

In general, a pharmaceutical composition according to the present invention can comprise various pharmaceutically acceptable excipients which will be selected depending on which functionality is to be achieved for the composition. A "pharmaceutically acceptable excipient" in the meaning of the present invention can be any substance used for the preparation of pharmaceutical dosage forms, including coating materials, film-forming materials, fillers, disintegrating agents, release-modifying materials, carrier materials, diluents, binding agents and other adjuvants. Typical pharmaceutically acceptable excipients include substances like sucrose, mannitol, sorbitol, starch and starch derivatives, lactose, and lubricating agents such as magnesium stearate, disintegrants and buffering agents.

The term "carrier" denotes pharmaceutically acceptable organic or inorganic carrier substances with which the active ingredient is combined to facilitate the application. Suitable pharmaceutically acceptable carriers include, for instance, water, salt solutions, alcohols, oils, preferably vegetable oils, polyethylene glycols, gelatin, lactose, amylose, magnesium stearate, surfactants, perfume oil, fatty acid monoglycerides and diglycerides, petroethral fatty acid esters, hydroxymethyl-cellulose, polyvinylpyrrolidone and the like. The pharmaceutical compositions can be sterilized and if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compound.

If liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavouring agents.

For parenteral application, particularly suitable vehicles consist of solutions, preferably oily or aqueous solutions, as well as suspensions, emulsions, or implants. Pharmaceutical formulations for parenteral administration are particularly preferred and include aqueous solutions in water-soluble form. Additionally, suspensions may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran.

Particularly preferred dosage forms are injectable preparations of a pharmaceutical composition of the present invention. Thus, sterile injectable aqueous or oleaginous suspensions can for example be formulated according to the known art using suitable dispersing agents, wetting agents and/or suspending agents. A sterile injectable preparation can also be a sterile injectable solution or suspension in a non-toxic parenterally acceptable diluent or solvent. Among the acceptable vehicles and solvents that can be used are water and isotonic sodium chloride solution. Sterile oils are also conventionally used as solvent or suspending medium.

Suppositories for rectal administration of a pharmaceutical composition of the present invention can be prepared by e.g. mixing the compound with a suitable non-irritating excipient such as cocoa butter, synthetic triglycerides and polyethylene glycols which are solid at room temperature but liquid at rectal temperature such that they will melt in the rectum and release the active agent from said suppositories.

For administration by inhalation, the pharmaceutical composition comprising a compound according to the present invention may be conveniently delivered in the form of an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Oral dosage forms may be liquid or solid and include e.g. tablets, troches, pills, capsules, powders, effervescent formulations, dragees and granules. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. The oral dosage forms may be formulated to ensure an immediate release of the active agent or a sustained release of the active agent.

### 3. Pharmaceutical compositions comprising nucleic-acid based inhibitors

The inhibitor of the present invention may be an antisense oligonucleotide targeting the GluN2D mRNA, a small interfering RNA (siRNA) targeting the GluN2D mRNA, a short hairpin RNA (shRNA) targeting the GluN2D mRNA, a microRNA (miRNA) targeting the GluN2D mRNA and a CRISPR-guide RNA (sgRNA) targeting GluN2D gene transcription. It may also be a vector encoding any of the afore-mentioned nucleic-acid based inhibitors.

Pharmaceutical compositions are formulated based on the mode of delivery / administration. Administration may be topical (e.g., by a transdermal patch), pulmonary, e.g., by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal or intramuscular injection or infusion; subdermal, e.g., via an implanted device; or intracranial, e.g., by intraparenchymal, intrathecal or intraventricular, administration. For example, compositions can be formulated for systemic administration via parenteral delivery, e.g., by intravenous (IV) delivery. For example, a composition provided herein (e.g., an LNP formulation) is formulated for intravenous delivery. Further, a composition provided herein (e.g., a composition comprising a GalNAc conjugate) is formulated for subcutaneous delivery.

The pharmaceutical compositions featured herein are administered in a dosage sufficient to inhibit GluN2D or mGluR2. In general, a suitable dose of antisense oligonucleotide, siRNA, shRNA, miRNA, or sgRNA (hereinafter "iRNA") will be in the range of 0.01 to 200.0 milligrams per kilogram body weight of the recipient per day, generally in the range of 1 to 50 mg per kilogram body weight per day. The pharmaceutical composition may be administered once daily, or as two, three, or more sub-doses at appropriate intervals throughout the day or even using continuous infusion or delivery through a controlled release formulation. In that case, the inhibitor contained in each sub-dose must be correspondingly smaller in order to achieve the total daily dosage. The dosage unit can also be compounded for delivery over several days, e.g., using a conventional sustained release formulation which provides sustained release of the inhibitor over a several day period. Sustained release formulations are well known in the art and are particularly useful for delivery of agents at a particular site, such as can be used with the agents of the present disclosure. In this embodiment, the dosage unit contains a corresponding multiple of the daily dose.

The effect of a single dose of an iRNA on GluN2D or mGluR2 levels can be long lasting. In a preferred embodiment, a single dose suffices for the treatment of a depressive episode. For example, the single dose may be formulated in a sustained release formulation that may last up to about 6 months. If used for relapse prevention, a preferred embodiment is the treatment with a twice-yearly administration of such a sustained release formulation. The skilled person will be aware of siRNA modifications and delivery technologies for providing such an long-lasting effect, which are also detailed in Hu et al. In another embodiment, several doses are administered for treatment of a depressive episode, such that subsequent doses are administered after an initial dose at, e.g., 3, 4, or 5-day or longer intervals, or at 1, 2, 3, or 4 week or longer intervals. Generally, longer intervals are preferred.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and in vivo half-lives for the individual inhibitor encompassed by the disclosure can be made using conventional methodologies or on the basis of in vivo testing using a suitable animal model.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful. Suitable topical formulations include those in which the inhibitors, in particular the iRNAs, are in admixture with a topical delivery agent such as lipids, liposomes, fatty acids, fatty acid esters, steroids, chelating agents and surfactants. Suitable lipids and liposomes include neutral (e.g., dioleoylphosphatidyl DOPE ethanolamine, dimyristoylphosphatidyl choline DMPC, distearoly-phosphatidyl choline) negative (e.g., dimyristoylphosphatidyl glycerol DMPG) and cationic (e.g., dioleoyltetramethylaminopropyl DOTAP and dioleoylphosphatidyl ethanolamine DOTMA). Inhibitors, e.g., iRNAs may be encapsulated within liposomes or may form complexes thereto, in particular to cationic liposomes. Alternatively, inhibitors, in particular the iRNAs, may be complexed to lipids, in particular to cationic lipids. Suitable fatty acids and esters include but are not limited to arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, di-laurin, glyceryl 1-monocaprate, I-dodecylazacycloheptan-2-one, an acylcarnitine, an acylcholine, or a Ci-20 alkyl ester (e.g., isopropylmyristate IPM), monoglyceride, diglyceride or pharmaceutically acceptable salt thereof. Topical formulations are described in detail in U.S. Patent No. 6,747,014.

There are many organized surfactant structures besides microemulsions that have been studied and used for the formulation of drugs. These include monolayers, micelles, bilayers and vesicles. Vesicles, such as liposomes, have attracted great interest because of their specificity and the duration of action they offer from the standpoint of drug delivery. As used in the present disclosure, the term "liposome" means a vesicle composed of amphiphilic lipids arranged in a spherical bilayer or bilayers.

In one embodiment, a GluN2D or mGluR2 inhibitor, in particular an iRNA, is fully encapsulated in the lipid formulation, e.g., to form a SPLP, pSPLP, SNALP, or other nucleic acid-lipid particle. As used herein, the term "SNALP" refers to a stable nucleic acid-lipid particle, including SPLP. As used herein, the term "SPLP" refers to a nucleic acid-lipid particle comprising plasmid DNA encapsulated within a lipid vesicle. SNALPs and SPLPs typically contain a cationic lipid, a non-cationic lipid, and a lipid that prevents aggregation of the particle (e.g., a PEG-lipid conjugate). SNALPs and SPLPs are extremely useful for systemic applications, as they exhibit extended circulation lifetimes following intravenous (i.v.) injection and accumulate at distal sites (e.g., sites physically separated from the administration site). SPLPs include "pSPLP," which include an encapsulated condensing agent-nucleic acid complex as set forth in PCT Publication No. WO 00/03683. The particles of the present disclosure typically have a mean diameter of about 50 nm to about 150 nm, more typically about 60 nm to about 130 nm, more typically about 70 nm to about 110 nm, most typically about 70 nm to about 90 nm, and are substantially nontoxic. In addition, the nucleic acids when present in the nucleic acid- lipid particles of the present disclosure are resistant in aqueous solution to degradation with a nuclease. Nucleic acid- lipid particles and their method of preparation are disclosed in, e.g., U.S. Patent Nos. 5,976,567; 5,981,501; 6,534,484; 6,586,410; 6,815,432; and PCT Publication No. WO 96/40964.

In one embodiment, the lipid to drug ratio (mass/mass ratio) (e.g., lipid to double-stranded RNA ratio) will be in the range of from about 1:1 to about 50:1, from about 1:1 to about 25:1, from about 3:1 to about 15:1, from about 4:1 to about 10:1, from about 5:1 to about 9:1, or about 6:1 to about 9:1.

The cationic lipid may be, for example, N,N-dioleyl-N,N-dimethylammonium chloride (DODAC), N,N-distearyl-N,N-dimethylammonium bromide (DDAB), N-(I -(2,3- dioleoyloxy)propyl)-N,N,N-trimethylammonium chloride (DOTAP), N-(I -(2,3- dioleyloxy)propyl)-N,N,N-trimethylammonium chloride (DOTMA), N,N-dimethyl-2,3- dioleyloxy)propylamine (DODMA), 1 ,2-DiLinoleyloxy-N,N-dimethylaminopropane (DLinDMA), I,2-Dilinolenyloxy-N,N-dimethylaminopropane (DLenDMA), 1,2- Dilinoleylcarbamoyloxy-3-dimethylaminopropane (DLin-C-DAP), 1 ,2-Dilinoleyoxy-3- (dime-thylamino)acetoxypropane (DLin-DAC), I,2-Dilinoleyoxy-3-morpholinopropane (DLin- MA), I,2-Dilinoleoyl-3-dimethylaminopropane (DLinDAP), I,2-Dilinoleylthio-3- dimethylaminopropane (DLin-S-DMA), I-Linoleoyl-2-linoleyloxy-3-dimethylaminopropane (DLin-2-DMAP), I,2-Dilinoleyloxy-3-trimethylaminopropane chloride salt (DLin-TMA.CI), 1,2-Dilinoleoyl-3-trimethyla-minopropane chloride salt (DLin-TAP.CI), I,2-Dilinoleyloxy-3-(N- methylpiperazino)propane (DLin-MPZ), or 3-(N,N-Dilinoleylamino)- 1,2-propanediol (DLinAP), 3-(N,N-Dioleylamino)-1,2-pro-panedio (DOAP), I,2-Dilinoleyloxo-3-(2-N,N- dimethylamino)ethoxypropane (DLin-EG-DM A) , I,2-Dilinolenyloxy-N,N - dimethylaminopropane (DLinDMA), 2,2-Dilinoleyl-4-dimethylaminomethyl- [1,3] -dioxolane (DLin-K-DMA) or analogs thereof, (3aR,5s,6aS)-N,N-dimethyl-2,2-di((9Z,12Z)-octadeca-9,12- dienyl)tetrahydro-3aH-cyclopenta[d][I,3]dioxol-5-amine (ALN100), (6Z,9Z,28Z,31Z)- heptatriaconta-6,9,28,31-tetraen-19-yl 4-(dimethylamino)butanoate (MC3), I,I'-(2-(4-(2-((2- (bis(2-hydroxydodecyl)amino)ethyl)(2-hydroxydodecyl)amino)ethyl)piperazin-I-yl)ethylazanediyl)didodecan-2-ol (Tech GI), or a mixture thereof. The cationic lipid may comprise from about 20 mol % to about 50 mol % or about 40 mol % of the total lipid present in the particle.

In another embodiment, the compound 2,2-Dihnoleyl-4-dimethylaminoethyl-[I,3]- dioxolane can be used to prepare lipid-siRNA nanoparticles. Synthesis of 2,2-Dilinoleyl-4- dimethylaminoethyl-[I,3]-dioxolane is described in United States provisional patent application number 61/107,998 filed on October 23, 2008.

In one embodiment, the lipid-siRNA particle includes 40% 2, 2-Dilinoleyl-4- dimethylaminoethyl-[I,3]-dioxolane: 10% DSPC: 40% Cholesterol: 10% PEG-C-DOMG (mole percent) with a particle size of 63.0 ± 20 nm and a 0.027 siRNA/Lipid Ratio.

The non-cationic lipid may be an anionic lipid or a neutral lipid including, but not limited to, dis-tearoylphosphatidylcholine (DSPC), dioleoylphosphatidylcholine (DOPC), dipalmitoylphosphati-dylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE), dioleoyl- phosphatidylethanolamine 4-(N-maleimidomethyl)-cyclohexane-I- carboxylate (DOPE- mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1 -trans PE, 1 -stearoyl-2-oleoyl-phosphatidy ethanolamine (SOPE), cholesterol, or a mixture thereof. The non-cationic lipid may be from about 5 mol % to about 90 mol %, about 10 mol %, or about 58 mol % if cholesterol is included, of the total lipid present in the particle.

The conjugated lipid that inhibits aggregation of particles may be, for example, a polyethyleneglycol (PEG)-lipid including, without limitation, a PEG-diacylglycerol (DAG), a PEG-dialky-loxypropyl (DAA), a PEG-phospholipid, a PEG-ceramide (Cer), or a mixture thereof. The PEG-DAA conjugate may be, for example, a PEG-dilauryloxypropyl (Cri), a PEG- dimyristyloxypropyl (Cri), a PEG-dipalmityloxypropyl (Cri), or a PEG- distearyloxypropyl (C]s). The conjugated lipid that prevents aggregation of particles may be from 0 mol % to about 20 mol % or about 2 mol % of the total lipid present in the particle.

In some embodiments, the nucleic acid-lipid particle further includes cholesterol at, e.g., about 10 mol % to about 60 mol % or about 48 mol % of the total lipid present in the particle. In some embodiments, the inhibitor, e.g. iRNA, is formulated in a lipid nanoparticle (LNP). LNP01

In one embodiment, the lipidoid ND98-4HC1 (MW 1487) (see U.S. Patent Application No. 12/056,230, filed 3/26/2008), Cholesterol (Sigma- Aldrich), and PEG-Ceramide C16 (Avanti Polar Lipids) can be used to prepare lipid-double stranded RNA nanoparticles (e.g., LNP01 particles). Stock solutions of each in ethanol can be prepared as follows: ND98, 133 mg/ml; Cholesterol, 25 mg/ml, PEG-Ceramide C16, 100 mg/ml. The ND98, Cholesterol, and PEG-Ceramide C16 stock solutions can then be combined in a, e.g., 42:48:10 molar ratio. The combined lipid solution can be mixed with aqueous double-stranded RNA (e.g., in sodium acetate pH 5) such that the final ethanol concentration is about 35-45% and the final sodium acetate concentration is about 100-300 mM. Lipid-double stranded RNA nanoparticles typically form spontaneously upon mixing. Depending on the desired particle size distribution, the resultant nanoparticle mixture can be extruded through a polycarbonate membrane (e.g., 100 nm cut-off) using, for example, a thermobarrel extruder, such as Lipex Extruder (Northern Lipids, Inc). In some cases, the extrusion step can be omitted. Ethanol removal and simultaneous buffer exchange can be accomplished by, for example, dialysis or tangential flow filtration. Buffer can be exchanged with, for example, phosphate buffered saline (PBS) at about pH 7, e.g., about pH 6.9, about pH 7.0, about pH 7.1, about pH 7.2, about pH 7.3, or about pH 7.4.

Preferred embodiments of the present application relate to:
1. A GluN2D inhibitor for use in the relapse prevention or treatment of a depressive episode as classified by ICD-10: F31-F34, wherein the GluN2D inhibitor is not R,S-ketamine or a pharmaceutically acceptable salt thereof, S-ketamine or a pharmaceutically acceptable salt thereof, or R-ketamine or a pharmaceutically acceptable salt thereof.
2. The GluN2D inhibitor for use according to embodiment 1, wherein the GluN2D inhibitor causes disinhibition and/or increases synaptic plasticity.
3. The GluN2D inhibitor for use according to embodiment 1 or 2, wherein the GluN2D inhibitor is selected from the group consisting of a small molecule targeting GluN2D, an antibody directed to GluN2D or an antigen-binding fragment thereof, an antisense oligonucleotide targeting the GluN2D mRNA, a small interfering RNA (siRNA) targeting the GluN2D mRNA, a short hairpin RNA (shRNA) targeting the GluN2D mRNA, a microRNA (miRNA) targeting the GluN2D mRNA and a CRISPR-guide RNA (sgRNA) targeting GluN2D gene transcription.
4. The GluN2D inhibitor for use according to embodiment 3, wherein the GluN2D inhibitor is an antisense oligonucleotide targeting the GluN2D mRNA, which is capable of binding to and/or is at least partially complementary to a region of the GluN2D gene or a regulatory element thereof.
5. The GluN2D inhibitor for use according to embodiment 3, wherein the GluN2D inhibitor is a siRNA targeting the GluN2D mRNA, which is capable of interfering with the gene expression of the GluN2D gene and comprises a first strand at least partially complementary to 15 nucleotides of the GluN2D gene, and a second strand of 15 to 30 nucleotides in length, wherein at least 12 nucleotides of the first strand and second strand are complementary to each other and form a siRNA duplex.
6. The GluN2D inhibitor for use according to embodiment 3, wherein the GluN2D inhibitor is a sgRNA targeting GluN2D gene transcription, which is at least partially complementary to 15 nucleotides of the GluN2D gene, and wherein in addition a CRISPR protein lacking endonuclease activity is administered, preferably the CRISPR protein is fused to at least a domain of Krüppel associated box (KRAB) protein.
7. An mGluR2 inhibitor for use in the relapse prevention or treatment of a depressive episode as classified by ICD-10: F31-F34, wherein the mGluR2 inhibitor is not 2R,6R-Hydroxynorketamine (HNK) or a pharmaceutically acceptable salt thereof.
8. The mGluR2 inhibitor for use according to embodiment 7, wherein the mGluR2 inhibitor causes disinhibition and/or increases synaptic plasticity.
9. The mGluR2 inhibitor for use according to embodiment 7 or 8, wherein the mGluR2 inhibitor is selected from the group consisting of a small molecule targeting mGLuR2, an antibody directed to mGluR2 or an antigen-binding fragment thereof, an antisense oligonucleotide targeting the mGluR2 mRNA, a small interfering RNA (siRNA) targeting the mGluR2 mRNA, a short hairpin RNA (shRNA) targeting the mGluR2 mRNA, a microRNA (miRNA) targeting the mGluR2 mRNA and a CRISPR-guide RNA (sgRNA) targeting mGluR2 gene transcription.
10. The mGluR2 inhibitor for use according to embodiment 9, wherein the mGluR2 inhibitor is an antisense oligonucleotide targeting the mGluR2 mRNA, which is capable of binding to and/or is at least partially complementary to a region of the mGluR2 gene or a regulatory element thereof.
11. The mGluR2 inhibitor for use according to embodiment 9, wherein the mGluR2 inhibitor is a siRNA targeting the mGluR2 mRNA, which is capable of interfering with the gene expression of the mGluR2 gene and comprises a first strand at least partially complementary to 15 nucleotides of the mGluR2 gene, and a second strand of 15 to 30 nucleotides in length, wherein at least 12 nucleotides of the first strand and second strand are complementary to each other and form a siRNA duplex.
12. The mGluR2 inhibitor for use according to embodiment 9, wherein the mGluR2 inhibitor is a sgRNA targeting mGluR2 gene transcription, which is at least partially complementary to 15 nucleotides of the mGluR2 gene, and wherein in addition a CRISPR protein lacking endonuclease activity is administered, preferably the CRISPR protein is fused to at least a domain of Krüppel associated box (KRAB) protein.
13. A vector encoding a GluN2D inhibitor selected from the group consisting of an antisense oligonucleotide targeting the GluN2D mRNA, a siRNA targeting the GluN2D mRNA, a shRNA targeting the GluN2D mRNA, a miRNA targeting the GluN2D mRNA and a sgRNA targeting GluN2D gene transcription or a mGluR2 inhibitor selected from the group consisting of an antisense oligonucleotide targeting the mGluR2 mRNA, a siRNA targeting the mGluR2 mRNA, a shRNA targeting the mGluR2 mRNA, a miRNA targeting the mGluR2 mRNA and a sgRNA targeting mGluR2 gene transcription for use in the treatment or relapse prevention of a depressive episode as classified by ICD-10: F31-F34.
14. A pharmaceutical composition comprising a GluN2D inhibitor, wherein the GluN2D inhibitor is not R,S-ketamine or a pharmaceutically acceptable salt thereof, S-ketamine or a pharmaceutically acceptable salt thereof, or R-ketamine or a pharmaceutically acceptable salt thereof, or a mGluR2 inhibitor, wherein the mGluR2 inhibitor is not 2R,6R-Hydroxynorketamine (HNK) or a pharmaceutically acceptable salt thereof, and a pharmaceutically acceptable excipient.
15. The pharmaceutical composition according to embodiment 14, wherein the GluN2D inhibitor is selected from the group consisting of an antisense oligonucleotide targeting the GluN2D mRNA, a siRNA targeting the GluN2D mRNA, a shRNA targeting the GluN2D mRNA, a miRNA targeting the GluN2D mRNA and a sgRNA targeting GluN2D gene transcription; and the mGluR2 inhibitor is selected from the group consisting of an antisense oligonucleotide targeting the mGluR2 mRNA, a siRNA targeting the mGluR2 mRNA, a shRNA targeting the mGluR2 mRNA, a miRNA targeting the mGluR2 mRNA and a sgRNA targeting mGluR2 gene transcription; and the pharmaceutically acceptable excipient comprises a lipid.

### 4. Examples

The following Examples are merely illustrative and shall describe the present invention in a further way. These Examples shall not be construed to limit the present invention thereto.

### Example 1: R,S-Ketamine in-vivo rehabilitates stress induced LTP-blockade

Intraperitoneal R,S-ketamine treatment (10mg/kg) significantly reduced immobility-time compared to control in mice subjected to the chronic despair model (CDM; see Serchov et al.; Holz et al.), a mouse model using forced swim sessions as stressor to induce a depressive-like state (Fig. 1A). Similarly, a single R,S-ketamine injection renormalized the reduced sucrose preference of CDM mice in the nose-poke sucrose preference test (see Holz et al.) (Fig. 1B).

In the next step, the effects of stress and R,S-ketamine treatment upon LTP- (long term potentiation) and LTD- (long term depression) inducibility in hippocampal brain slices of mice were investigated. Electrical stimulations of Shaffer collaterals, resulting in excitatory postsynaptic potentials (EPSPs), were paired with postsynaptic action potential (AP) inductions in CA1 neurons. 125 of these EPSP→AP pairings formed the associative LTP (aLTP / associative long term potentiation) protocol and resulted in a stable increase of EPSP-amplitude (Fig. 1C), whilst 360 AP→EPSP pairings formed the associative LTD (aLTD / associative long term depression) protocol and resulted in a decrease of EPSPS amplitude (Fig. 1C). In CDM mice, LTP induction was completely abolished whilst LTD was facilitated, which alterations were fully restored by R,S-ketamine treatment (Fig. 1 D,E). This confirms that R,S-ketamine is able to reverse LTP-and LTD-effects.

To address the underlying mechanism and the question whether either or both of LTD blockade and LTP rehabilitation are causal for antidepressive potency, R,S-ketamine treatment was injected shortly before each swim session in the CDM, and aLTP and aLTD were assessed afterwards. Such R,S-ketamine treatment prevented the stress-induced aLTP blockade, but had a weaker effect on the aLTD facilitation (Fig. 1F), indicating that the NMDAR antagonist R,S-ketamine predominantly modulates LTP.

### Example 2: Interneurons play key role in the modulation of plasticity by ketamine

To further elaborate on the underlying mechanism, either ketamine consisting of the two enantiomers S- and R-ketamine (K), or consisting only of S-ketamine (SK) was applied to brain slices of unstressed mice in a bathing solution. Both K and SK resulted in complete inhibition of LTP in slices of non-stressed animals (Fig. 2A). Similar results were obtained using tetanic 100Hz stimulation of Schaffer collaterals, a homosynaptic form of synaptic plasticity (Fig 2B). NMDAR-independent aLTD was unaffected by R,S-ketamine and SK addition to the bath (Fig. 2C), whereas NMDAR-dependent 1Hz LTD was fully blocked in this condition (Fig. 2D).

While the bathing solution applied to non-stressed animals contains the GABA_{A} antagonist picrotoxin (PIC), injections given to CMD mice did not. The presence/absence of PIC results in a deactivated/functional state of inhibiting GABAergic interneurons, respectively. To elucidate the role of interneurons, these experiments in brain slices of unstressed mice were repeated in the absence of PIC. Surprisingly, neither K nor SK blocked aLTP induction in the absence of PIC (Fig.2E) and LTD induction could be blocked by K, but not SK (Fig. 2F). PIC alone did not change aLTP induction (data not shown).

These results show that in the presence of functional interneurons, K and SK no longer inhibit LTP in concentrations estimated to be equivalent of therapeutic use. Under these conditions R,S-ketamine might inhibit tonically active NMDAR on interneurons in addition to voltage-dependent postsynaptic NMDAR in the synaptic cleft. The subunit composition of NMDARs on interneurons in general is different from synaptic NMDARs, with a higher expression of GluN2D subunits on interneurons. R,S-ketamine has a higher affinity for NMDAR containing GluN2D subunit, which might explain a preferential binding to NMDARs on interneurons, causing disinhibition of presynaptic neurons (see Monyer et al.; Perszyk et al.; Vyklicky et al.; Traynelis et al.). This disinhibition is thought to increase glutamate release into the synaptic cleft. Following this hypothesis, a low-dose R,S-ketamine treatment should lead to more glutamate in the synaptic cleft and therefore not only rehabilitate plasticity in depressed animals, but also enhance LTP in healthy animals. To address this question, the aLTP protocol needed to be modified, since it suffers from a ceiling effect (a potential further enhancement of transmission would otherwise not be detectable). Therefore the number of EPSP→AP pairings was reduced from 125 as in aLTP to 25 in the low intensity LTP protocol (liLTP). This resulted in a smaller, but stable LTP in the absence of PIC (Fig 2G). Under these conditions, R,S-ketamine application showed a concentration depending effect; a reduced concentration of 5 µmol/l R,S-ketamine caused a significant increase in EPSP amplitude compared to controls, whereas a higher concentration of 10 µmol/l blocked LTP induction (Fig 2H).

Taken together, our results suggest a decisive role of preferential binding of R,S-ketamine to interneurons for its augmentation of LTP. In other words, the binding to the NMDARs of interneurons is decisive, wherein the NMDARs of interneurons differ in their subunit composition from synaptic NMDARs. This was investigated in more detail in the following Examples.

### Example 3: Modulation of GluN2D exerts antidepressive potency

GluN2D is almost exclusively expressed on inhibiting interneurons. To address whether modulation of GluN2D might selectively enhance plasticity and execute antidepressive potency, the specific GluN2C/D antagonist NAB-14 (see Yao et al.; Swanger et al.) was tested. NAB-14 did not change synaptic transmission (Fig. 3A,B), but increased liLTP (Fig. 3C) and strongly increased the EPSP amplitude after aLTD stimulation (Fig. 3D), when applied to brain slices of unstressed mice in a bathing solution. Though NAB-14 was described as a specific GluN2C/D antagonist, the GluN2C subtype is of no relevance in the present setup, since it is neither present on interneurons nor on pyramidal cells in the hippocampus (see Ravikrishnan et al.).

Furthermore, the stress-induced LTP blockade in DM mice could be partially (5 mg/kg) and completely (10 mg/kg) reversed by NAB-14 *in vivo* application (Fig. 3E, H).

In order to differentiate between an increase of EPSP-amplitude by excitation or by plastic changes, EPSP-morphology was analyzed. A decrease of EPSP-slope and an increase of EPSP decay found in CDM could effectively be reversed by previous NAB-14 treatment (Fig. 3F, G). To verify the proposed mechanism via GluN2D, the positive allosteric modulator CIQ (a potentiator of NMDA receptors containing GluN2C/GluN2D) and the GABA_{A} agonist lorazepam (see Kaila) were injected into CMD mice 20 min. prior to NAB-14 treatment. Both prevented antidepressive action of NAB-14 by abolishing LTP reestablishment (Fig. 3H). Similarly, immobility time and sucrose preference of CDM mice treated with NAB-14 did not improve when CIQ or lorazepam were applied prior to NAB-14 (Fig. 3I, J). General locomotor and exploratory activity were not significantly affected in the open field test by either NAB-14 or the carrier solution alone (data not shown).

To experimentally confirm the hypothesis that the differential blockade of NMDARs on Interneurons occurs within the investigated conditions (drug concentration, time frame etc.), NMDAR-currents were directly measured in CA1 pyramidal cells and also in fluorescent-labelled somato-statin positive Interneurons (SOM). The mean amplitude of NMDAR inward currents in SOM was significantly reduced by the wash-in of NAB-14, whilst no relevant NMDAR reduction occurred on CA1 pyramidal cells. In addition, R,S-ketamine application to the bathing solution reduced NMDAR currents in SOM significantly stronger than in CA1 pyramidal cells (Fig. 3K). These results indicate a functionally stronger blockade of NMDAR currents in SOM by NAB-14 and R,S-ketamine, whilst R,S-ketamine leads to a stronger reduction in CA1 than NAB-14. These finding are concordant with previous works by others showing that GluN2D is almost exclusively expressed on interneurons and that NAB-14 exerts a highly selective blockade of GluN2D. R,S-Ketamine on the other hand also shows relevant (but lower) affinities to NMDAR subunits other than GluN2D, therefore also significantly blocking NMDAR currents in CA1 cells, where other subunits are expressed. The disinhibiting effect of R,S-ketamine is represented as the difference between both currents (SOM vs. CA1).

To further confirm that GluN2D modulation can exert antidepressive potency, GluN2D siRNA (50 nmole/animal) was injected intrathecally using IN-VIVO-JET-PEI as the carrier substance, following a recently established protocol (see Njoo et al.). Injection was performed the day after the induction phase of CDM and animals were left in their home cages to rest for 3 days. The evaluation of immobility time (Fig. 4A), locomotor activity (Fig. 4B) and LTP inducibility (Fig. 4C,D) mirrored results obtained with NAB-14, confirming GluN2D to be the critical modulation point. Real-time PCR was used to verify RNA downregulation in hippocampus and frontal cortex (Fig. 4E) and Western Blot to shown protein downregulation (4F,G) of GluN2D.

Summarizing these results, R,S-ketamine's restorative effects depend on the integrity of interneurons. Further, the NMDAR subunit GluN2D, which is mainly expressed on tonically active, inhibiting interneurons, plays a key role. The narrow therapeutic window between the antidepressive effect and the beginning of anesthesia could be explained by the higher affinity to GluN2D compared to other NMDAR subunits. In other words, low dose R,S-ketamine preferentially binds to GluN2D subunits and results in a functional disinhibition of pyramidal cells leading to glutamate surge in the synaptic cleft → enhanced information processing; antidepressive effect (Fig. 5). This effects is overwhelmed at a higher R,S-ketamine concentration by the blockade of synaptic NMDARs → reduced/blocked information processing; anesthetic effect (Fig. 5). NAB-14 exerts antidepressive potency that can be blocked by CIQ (10mg/kg) and lorazepam (0,125mg/kg) via modulation of GluN2D, and GluN2D can be targeted for antidepressive effects by siRNA. Due to the almost exclusive expression on interneurons in adults, GluN2D qualifies as treatment target, with high hopes of low side effects. For instance, NAB-14 did not show side effects in in vivo experiments.

Thus, while it was known that R,S-ketamine has an antidepressive effect at low dose, it was thus far not known that this antidepressive effect is mediated via its binding to the GluN2D subunit of NMDAR. Having established this link, the inventors went on to directly inhibit GluN2D by using a small molecule (NAB-14) or siRNA, and also observed the antidepressive effect in the experimental models that were used.

### Example 4: Modulation of mGluR2 rescues stress-induced impairment of synaptic plasticity

Metabotrophic glutamate receptor 2 (mGluR2) is predominantly expressed in presynaptic neurons and functionally acts as an inhibiting autoreceptor. It has been hypothesized that the R,S-ketamine metabolite 2R,6R Hydroxynorketamine (HNK) exerts antidepressant-like activity via a blockade of mGluR2 (see Zanos et al.), resulting in a disinhibition of presynaptic glutamate release.

To assess whether inhibition of mGluR2 leads to a disinhibition of presynaptic cells, mGluR2 antagonist 1 (which is commercially available and corresponds to 7-[(2,5-dioxopyrrolidin-1-yl)methyl]-4-(4-fluorophenyl)quinoline-2-carboxamide), which is specific for mGluR2, was tested in mouse hippocampal brain slices similar to GluN2D examples above. Healthy animals show essentially no reduction in amplitude of EPSPs after aLTD protocol, while CDM mice displayed a significantly reduced mean EPSP amplitude. This facilitated inducibility of long-term depression represents an electrophysiological correlate of a depressive-like state in rodents. Treatment with either HNK or mGluR2 antagonist 1 abolishes this reduction of amplitude in CDM mice, showing an antidepressive-like effect (Fig. 6A).

When treatment with HNK or mGluR2 antagonist 1 occurred prior to swim sessions, even greater amplitudes after aLTD exceeding those observed in healthy animals were observed (Fig. 6B) in male and female mice.

Mean EPSP amplitude after aLTP induction was significantly reduced in CDM mice compared to non-stressed mice, indicating a depressive-like effect. This blockade of LTP inducibility could be prevented neither by pretreatment with HNK nor b pretreatment with mGluR2antagonist 1 (Fig. 6C).

These data indicate that HNK anti-depressive-like effects are indeed mediated via mGluR2 on presynaptic neurons. Like GluN2D, mGluR2 is an attractive target for disinhibition by antagonists or siRNA due to selective expression, which should result in few side effects.

### Example 5: Ketamine blocks NMDAR currents in postsynaptic interneurons and converts subthreshold EPSPs to APs.

Next, the functional consequences of the EPSP increases as a result of GluN2D inhibition observed in Examples 1-3 were tested.

In previous experiments, R,S-ketamine was shown to decrease the spontaneous inhibitory postsynaptic current (IPSC) frequency and amplitude in CA1 and medial prefrontal cortex (mPFC) pyramidal cells (PCs) and to increase the probability of conversion of subthreshold EPSPs to APs (Wideman & McMahon; Gerhard et al.). We confirmed these findings by a feedback-loop activity protocol (FLAP). To activate the SOM-dependent feedback loop, we injected a depolarizing current into the soma of CA1 PCs, evoking a burst of APs. Subsequently, an EPSP was induced by Schaffer collateral stimulation, which served as a readout for the effects of the modulation of the excitation/inhibition balance on glutamatergic transmission in the dendrites of the PC (Fig. 7 A). After achieving a stable baseline for 10 min, R,S-ketamine (10 µM) was added to the bath solution. This dramatically increased the EPSP amplitude (Fig. 7 B), most likely by specific inhibition of the feedback loop at the CA1-SOM synapse described in Example 3 (Fig. 5). The increase of the EPSPs-amplitude by ketamine resulted in a conversion of the EPSPs to APs, indicated by a higher percentage of APs after the addition of ketamine to the bathing solution (Fig. 7 C). That is, these results confirm that R,S-ketamine increases the probability of conversion of subthreshold EPSPs to APs, i.e. functionally disinhibits pyramidal cells.

In the model of Fig. 5, disinhibition of pyramidal cells leads to a glutamate surge in the synaptic cleft, which in turn leads to enhanced information processing and antidepressive effect. To assess the putative role of such an increased glutamate release in the modulation of LTP by R,S-ketamine, we determined the paired-pulse ratio (PPR) before and after induction of li-LTP (compare Fig. 2H). The change in the ratio of the amplitudes of two consecutive EPSPs (50 ms interval) indicates an altered presynaptic release probability. The PPR was unchanged in both the absence and the presence of 5 or 10 µM ketamine (Fig. 7D), indicating a postsynaptic effect in line with the model of Fig. 5.

Next, the earlier described results describing the effect of R,S-ketamine on NMDAR currents (compare Fig. 3K) were expanded to greater detail. R,S-ketamine (10 µM) reduced the amplitude of isolated NMDAR currents in PCs, as observed in previous Examples. Under the same experimental conditions, NMDAR currents were evoked in oriens-lacunosum/moleculare SOMs, which were identified by morphological and electrophysiological characteristics and fluorescence excitation in SOM-Cre (SST tm2.1(cre)Zjh/J)) td-Tomato mice (see Fig. 8 G). The R,S-ketamine-induced reduction in the NMDAR current amplitude was significantly more pronounced in SOMs than in PCs (example trace Fig. 7 E, cumulative analysis Fig. 7 F), in line with the observations of Example 3 and Fig. 3K. Again, this is concordant with the fact that GluN2D is almost exclusively expressed on interneurons, such that SOMs should respond more strongly to R,S-ketamine. Because R,S-ketamine also shows relevant (but lower) affinities to NMDAR subunits other than GluN2D, there is still significant, but lower, blocking NMDAR currents in pyramidal cells, where other subunits are expressed.

In summary, these data demonstrate in detail that the functional consequence of R,S-ketamine induced EPSP increase is increased conversion to post-synaptic APs via glutamate surge in the synaptic cleft.

### Example 6: Specific GluN2D blockade on SOM Interneurons increases EPSPs and postsynaptic AP probability.

To further describe the role of GluN2D in shaping EPSPs, NAB-14, which is specific for GluN2D and does not exhibit the lower but relevant affinities to the other NMDAR subunits that R,S-ketamine has, was used in the FLAP protocol in the same experimental set-up previously described for R,S-ketamine (Example 5, Fig. 7 A). The addition of NAB-14 to the bath solution, just as for R,S-ketamine, significantly increased the EPSP amplitude, most likely by specific inhibition of the feedback loop at the CA1-SOM synapse. PC excitability, as assessed by the number of APs during the burst and input resistance (Rm), did not significantly change (Fig. 8 A). When NAB-14 was applied in the presence of the GABA_{A} positive allosteric modulator diazepam (compared to lorazepam in Example 3), the increase in the EPSP amplitude was abolished (Fig. 8 B), confirming GluN2D dependence of the mechanism. Coefficient of variation (CV) analysis of EPSP slopes before and after wash-in of NAB-14 revealed results that were consistent with a postsynaptic mechanism (Fig. 8 C).

In the absence of AP bursts, i.e., without targeted activation of the feedback loop, neither NAB-14 nor diazepam wash-in significantly altered the EPSP amplitude (Fig. 8 D). These results suggest GluN2D antagonism leads to specific inhibition of the SOM-dependent feedback loop of Fig. 5, resulting in a decreased inhibition/excitation (I/E) balance.

This change in I/E resulted in a conversion of EPSPs to APs in FLAP by NAB-14 presence in the bathing solution (Fig. 8 E). Furthermore, NAB-14 was shown to reduce the amplitude of NMDAR currents in SOMs but not in CA1 PCs (Fig. 8 F), suggesting that it had even higher selectivity than ketamine (compare Fig. 7 F). This is again consistent with the observations of Example 3 and Fig. 3K. Since GluN2D, the specific target of NAB-14, is almost exclusively expressed on interneurons and not on CA1 PCs, NMDAR currents are only blocked in SOMs.

In order to prove that the experimental set-up used in the above Examples specifically addressed controls, experiments were performed showing that currents at -80 mV could be fully blocked by α-amino-3-hydroxy-5-methyl-4-isoxazolepropionic acid receptor (AMPAR) specific blocker CNQX (Honoré et al.) at 10 µM. Addition of DAPV (highly specific NMDAR blocker; Lodge et al.) did not significantly change the measured current, clearly showing that, as expected since NMDAR is physiologically blocked by Mg²⁺, currents were driven by AMPAR activation by glutamate (Fig. 8 H). At a holding potential of +40 mV, elicited currents could only partially be reduced by CNQX, whilst DAPV resulted in a full blockade. This indicates that, in the condition used in Fig. 7 E, F and Fig. 8 F (+40 mV under the presence of CNQX), the measured current is purely driven by NMDAR activity.

In summary, these results further demonstrate that the antidepressive effect of R,S-ketamine is mediated via GluN2D, which NAB-14 exclusively inhibits in these experimental conditions, and the functional consequence of EPSP increases mediated via GluN2D inhibition is increased conversion to post-synaptic APs via glutamate surge in the synaptic cleft.

### Example 7: QNZ46 also exerts an antidepressive effect.

To provide further evidence that it is in fact the GluN2D subunit that plays the decisive role in antidepressive response, the specific GluN2D blocker QNZ46 (Hansen & Traynelis; Mosley et al.) previously used, but not tested for an antidepressive effect, in Zhang et al. was used in the CDM (Vestring et al.) model. In line with the results of Example 3 for the structurally unrelated compound NAB-14 and anti-GluN2D siRNA (Figs. 3 I and 4 A, respectively), and contrary to the hypothesis of Zhang et al. (and Ide et al.) that GluN2D agonists (and not antagonists) would be required to treat depression, QNZ46 significantly reduced immobility time in CDM swim test (Fig. 9 A). These data further confirm that it is specific GluN2D inhibition that exerts an antidepressive effect.

### Experimental procedures and materials used in the above examples

### Slice preparation

Animals were preoxygenated in a 100% oxygen atmosphere for 5 min before cervical dislocation and decapitated according to national and institutional guidelines. Transverse 300 µM-thick-slices were cut from hippocampus with a Vibratome (VT1200, Leica, Japan). Slices were prepared in artificial cerebrospinal fluid (ACSF) containing (in mmol/l): 125 NaCl, 25 NaHCO3, 1.25 NaH2PO4, 2.5 KCL, 1 MgCl2, 27 Glucose, 2 CaCl2 bubbled with carbogen (95% O2, 5%CO2).

### Electrophysiology

After 20 min resting time at 35°C, slices were kept at room temperature in aCSF and transferred into the recording chamber (volume approx. 2-3 ml) and continuously superfused with aCSF (rate approx. 5-10m1/min⁻¹). Differential interference contrast video microscopy was used to identify location of CA1 pyramidal neurons (Zeiss Axioskop 2 FS plus, Zeiss Microscopy, Germany). Beside optical identification neurons were classified according to their characteristic firing frequency adaptation to long depolarising current pulses. Borosilicate glass tubes (2.0 mm outer diameter, 0.5 mm wall thickness; Hilgenberg, Germany) were used to pull patch pipettes. Pipettes with a resistance of 5 - 10 MΩ were used and series resistance of 10 - 50 MΩ was compensated by bridge balance. Patch pipettes were filled with an internal solution containing (in mM) for EPSP-measurements: 132K-gluconate, 20KCL, 2MgCl2, 10Hepes, 0.1EGTA, 4 Na2ATP, 0.3NaGTP. PH was adjusted to 7.2 with KOH. For current measurements: 135Csglu-conate, 2CsCl, 5 QX314, 10 Hepes, 10EGTA, 2MgCl₂, 2Na₂ATP, 2TEACI, pH was adjusted to 7.2 with HCl. Osmolarity (280-300 mosmol/l) was controlled at the beginning of each experimental day with Osmomat (Gonotec GmbH, Germany).

A stimulation pipette (patch pipette with 1 - 3 MΩ resistance when filled with internal solution) was placed superficially in the stratum radiatum of the CA1 region in approximately 30-50 µM distance to the pyramidal cell layer. Subthreshold excitatory postsynaptic potentials (EPSPs, 2 - 7 mV) were evoked by Schaffer collateral stimulation with voltage pulses of 10-80 V (frequency of 0.1 Hz, duration of 200 µs) using a stimulus isolator (Model 2100 Isolated pulse stimulator, Carlsborg, U.S.A.). Resting membrane potentials were between -75 and -65 mV and holding potential was -70 mV. 50 ms current test pulses (leading to 5 -10 mV hyperpolarization) were applied to control input and series resistances after every 10th EPSP. EPSPs were combined with APs triggered by short (3 ms) current application of 900 pA via the patch-clamp electrode. EPC-10 amplifiers (HEKA, Germany) were used and signals were filtered at 5 kHz. Patchmaster NEXT software (Version 1.2, HEKA, Germany) was used for data acquisition and experiments were performed at room temperature.

Experiments were discarded if (i) the series resistance (RS) changed by more than 30% during the course of the experiment, (ii) evidence of ictal discharge was observed, (iii) the membrane potential between start and end of the experiment differed by more than 5mV, or (iv) if the neurons did not respond to a firing pattern control pulse at the beginning and at end of the experiments.

### LTP induction protocols/wash-in experiments in current-clamp recordings

aLTP: Five EPSPs and five postsynaptic APs were paired at 100 Hz with a 5 ms delay (AP after EPSP). Five of these bursts of synchronized EPSP→AP pairs were applied at theta frequency (5 Hz), followed by an interval of 10 sec and 4 more theta blocks, resulting in 125 EPSP/AP pairings.

liLTP: In contrast to aLTP, only one theta block was applied, resulting in 25 EPSP/AP pairings. 100Hz LTP: EPSPs were elicited at a frequency of 100 Hz for 4 x 1 s with an interval of 2 sec between each tetanus.

aLTD: 360 postsynaptic APs were pared with 360 EPSPs with a delay of 20-30 ms between each AP/EPSP pairing at a frequency of 1Hz.

1Hz LTD: 600 EPSPs were elicited at a frequency of 1Hz whilst CA1 pyramidal cell membrane potential was hold at -60mV.

Wash-in experiments: EPSPs were elicited at a frequency of 0.1 Hz. The mean EPSP amplitude was calculated at 0 - 5 min as baseline. Substances were applied in the bathing solution after the baseline for 30 min; mean EPSP amplitudes were calculated between 25-30 min after wash-in.

### NMDA-currents in voltage-clamp recordings

From a holding potential of -70 mV cells were depolarized to +40 mV for 3 s and an EPSP was induced by Schaffer collateral stimulation 2.5 s after the beginning of the depolarization. 10 EPSPs with an interval of 30 s were averaged before and after wash-in of ketamine, respectively. τ0, peak amplitude and the area under the curve (AUC) were fitted either with Stimfit software (Version 0.11.9, United Kingdom) or with Patchmaster NEXT software.

### Identification of SOM interneurons

Slices from mice expressing Td Tomato in SOM interneurons (SOM-Cre (SST tm2.1(cre)Zjh/J))) or wildtype mice were prepared as described above. Interneurons of the feedback-loop (mainly SOM interneurons) could visually be identified by elucidating (ex553/em580) fluorescence or by their morphologic characteristics and their location in the stratum oriens. EPSPs were evoked by placing a stimulation electrode into the stratum oriens in a distance of the measured cell of approx. 200 µm. Stimulation strength was reduced to ≤ 10 µA.

### Immunoblotting

Hippocampal tissue was dissociated in ice-cold RIPA buffer (30 mM Tris Base, pH 7.4, 150 mM NaCl, and 1 % Triton X-100) containing protease and phosphatase inhibitors. Debris was removed by centrifugation (13,000 × g at 4°C, 15 min). Protein quantification was performed according to the BCA method (Pierce). Proteins (50 µg) were resolved on 10 % polyacrylamide gel under denaturing conditions and transferred onto a polyvinylidene difluoride (PVDF) membrane. Membranes were blocked with Tris-buffered saline (10 mM Tris and 200 mM NaCl, pH 7.4) containing 5 % nonfat dry milk. Blots were incubated overnight with the GluN2D primary antibody from Lifespan Biologicals (LS-C120005) (1:500). After incubation with the appropriate anti-rabbit IRDye 800CW secondary antibody, proteins were visualized with the Odyssey^{®} Imaging System. Tubulin (Abcam; ab11321; 1:15 000) was used as loading control and ImageJ (https://imagej.nih.gov/ij/) was used to calculate the relative quantification of the bands.

### Real-time PCR

RNA was isolated from powdered frozen hippocampal samples using the NucleoSpin RNA kit (Machery Nagel) and cDNA was prepared using Oligo d(T) primers and Ready-To-Go You-Prime First-Strand Beads (GE Healthcare). Real-time PCR was performed using the Takyon No Rox SYBR MasterMix dTTP Blue Kit (Eurogentec) using a LightCycler 480 (Roche). As internal control genes, the reference genes GAPDH (Glycerinaldehyd-3-phosphat-Dehydrogenase) and RPS18 (40S ribosomal protein S18) were used. The following primer pairs (sequences provided as 5'-3') were used:
*GluN2D* (fwd CTGTGTGGGTGATGATGTTCGT (SEQ ID NO: 5), rev
GTGAAGGTAGAGCCTCCGGG (SEQ ID NO: 6))
*GAPDH* (fwd ACAACTTTGGTATCGTGGAAGG (SEQ ID NO: 7), rev GCCATCACGCCACAG-TTTC (SEQ ID NO: 8))
*RPS18* (fwd GCGGCGGAAAATAGCCTTTG (SEQ ID NO: 9), rev GATCACACGTTCCAC-CTCATC (SEQ ID NO: 10))

Amplification was performed with an initial denaturation of 45 cycles of 95 °C for 10 s, followed by 45 cycles of 60 °C for 15 s and 72 °C for 15 s. A melting curve was obtained at the end of cycling to verify the amplification of a single PCR product. The expression of the *GluN2D* gene relative to a normalization factor (geometric mean of two reference genes) was calculated using the 2^{-ΔCₜ} method as previously described (Schmittgen and Livak, 2008).

### SiRNA application

2 hours after the induction phase of the CDM, animals were anaesthetized with Isoflurane and positioned on a heating mat. The lower back was shaved, disinfected and siRNA compound injected slowly in the groove between L5 and L6 vertebrae column. SIRNA compound contained (per animal): 0.06µl IN-VIVO-JET-PEI solution, 50nmole siRNA, 5µl Glucose solution (10%) and 4.94 µl H₂O. Afterwards animals rested for 3 days in their cages before the outcome was assessed.

### Chronic despair model (CDM)

Induction phase: Mice were forced to swim in a glass cylinder (Ø 26 cm, 60 cm high) filled to 25 cm with 25 °C warm water for 10 min on 5 consecutive days. After the swimming, the animals were kept in their cages for two days before treatment and experiment readout was performed. The immobility time was measured in each session.

Behavioral Readout: After the treatment intervention, animals were either tested in an additional swim session (test day) to measure immobility time or tested in the InteliCage using the Nosepokes sucrose preference test. For the assessment of the immobility time, mice were vide-otaped and two independent raters who were blinded to the experimental group analyzed the videos. Immobility time was defined as the cumulative time that the animals spend stationary with only enough movements of the tails or the forepaws to keep the head above the water surface. No distance is actively travelled except for passive floating, no directed movement of the front paws observed and the body of the animal mostly oriented parallel to the walls of the cylinder.

### Electrophysiological Readout

Long-term plasticity or molecular analyses were assessed as described above after the induction phase of the CDM and the specific treatment.

### Open Field Test (OFT)

The test was performed in a square arena (50 × 50 cm) surrounded by a 35 cm high wall made of gray PVC. Mice were placed in the center of the field and allowed to move freely. Behavior was recorded for 10 min and total distance traveled was analyzed.

### Nose poke sucrose preference test

The IntelliCage system (TSE Systems) allows simultaneous analysis of spontaneous and exploratory behavior, activity pattern, and drinking preference of up to 16 group-housed mice implanted with radio-frequency identification (RFID) transponders. The unit consists of an open common space with 4 red shelters in the center and 4 recording corners. Mice have free access to food in the middle of the IntelliCage, water is available in the corners behind remote-controlled guillotine doors. Each corner is equipped with 2 drinking bottles and permits the visit of only one mouse at the same time. The scored parameters - the nosepokes toward the doors and the licks on the bottles - were monitored by a PC-based tracking software (IntelliCage Plus, TSE Systems). Initially, the mice were allowed to adapt to the IntelliCage for at least 7 days with water available ad libitum in all corners. Then for 3 days the animals were habituated to the sucrose taste: in each corner one of the bottles was filled with 1% sucrose solution and the other one with water. Both doors in the corner were open allowing free choice between the bottles. Next, a nosepoke adaptation period was carried out, where all doors were closed and the mice had to perform a nosepoke to open them. The opened door closes automatically after 5 s of drinking. In all tasks involving sucrose-filled bottles, the positions of the bottles were exchanged every 24h. The Nosepoke SPT protocol was used (see Alboni et al.) for measurement of sucrose preference with gradually increasing effort (number of nosepokes) to reach the sucrose bottles for a short period of time (12 h). In this paradigm each door opens in response to a nosepoke and closes after 5 s licking. The number of nosepokes needed to open a door to a side with a sucrose containing bottle gradually increases (1, 2, 3, 4, 5, 6, 8, 10, 12, 16, 20, 24) after every 8 sucrose licking sessions. For each bottle the number of licks was recorded and the averaged sucrose preference was calculated as percentage of the total number of licks.

### Data analysis and statistics

All given values are mean ± SEM and error bars represent SEM in figures. A maximum of two hippocampal slices per animal were used, n represent the number of experiments. For statistical analysis GraphPad Prism Version 8.3.0 (GraphPad Software, USA) was used. Two blinded raters analyzed the CDM behavioral readout, in all other experiments, neither randomization nor blinding was used.

EPSP measurement: For baseline measurement, the mean amplitudes of 30 consecutive EPSPs before the induction protocol were calculated. 25 min after LTP induction the mean amplitude of 30 consecutive EPSPs was calculated and compared to the baseline of the same measurement. Changes of EPSP amplitudes were expressed as percentages of the baseline measurement and analysed for each experimental group applying a two-tailed Wilcoxon test. The differences between separate experimental groups were assessed using the Mann-Whitney test, not assuming normal distribution. Assuming a standard deviation of 25% of EPSP amplitudes, a sample size of 5 per group is sufficient to reveal a group difference of 50 % (two-tailed alpha 0.05, power 0.8).

### Chemicals

R,S--Ketamine hydrochloride, S-Ketamine hydrochloride were purchased from Sigma-Aldrich (Germany). Picrotoxin, NAB-14 were purchased from Tocris (UK) and GluN2D siRNA from Thermo fisher scientific (USA). Diazepam was purchased from Sigma Aldrich (via Merck) and dissolved in DMSO (Dimethyl sulfoxide) and added to ACSF (1% DMSO for total concentration). QNZ-46 (4-[6-Methoxy-2-[(1E)-2-(3-nitrophenyl)ethenyl]-4-oxo-3(4H)-quinazolinyl]-Benzoic acid) was purchased from Sigma Aldrich (via Merck) and dissolved in DMSO and NaCl (0.9%). Animals received intraperitoneal injections two hours before behavioural testing. CNQX (6-Cyano-7-nitroquinoxaline-2,3-dione) and DAPV (D-(-)-2-Amino-5-phosphonopentanoic acid) were purchased from Tocris and dissolved in aCSF. All other chemicals were obtained either from Sigma-Aldrich or Tocris. Stock solutions were prepared in distilled water. Agents were either injected or applied in the bath solution.

### FLAP

A 300 ms depolarizing current between +100 and +600 pA was injected via the patch pipette into a PC to elicit a train of 5-10 APs. After an interval of 600 ms, one EPSP (5-10 mV amplitude) was elicited by Schaffer collateral stimulation. This pattern was continuously repeated at a frequency of 0.2 Hz. Substances were added by bath application after a stable 10 min baseline recording for an additional 20 min. For data analysis the maximum EPSP amplitudes from minutes 0-10 were averaged and compared to averaged maximum EPSP amplitudes from minutes 20-30.

### CV analysis

The slope of the EPSP rise at 20% to 40% of its maximal amplitude, at which point an approximately linear increase in its voltage could be assumed, was fitted with Fitmaster software (HEKA, Germany). We analyzed 20 EPSPs before and 20 EPSPs 25-30 minutes after NAB-14 wash-in in the FLAP protocol for each experiment. The coefficient of variation (CV) is the standard deviation of the EPSP slopes divided by the mean. The inverse square of the CV of the postwash-in slopes was divided by the inverse square of the prewash-in slopes and plotted against the corresponding normalized slopes. Pre- and postwash-in measurements from a single experiment are connected by a line. In a standard quantal model for synaptic transmission, synaptic responses are affected either by presynaptic changes in the number of release sites and/or the probability of release or by modifications of the postsynaptic response to a single vehicle. A change in the ratio of CV-2 reflects a presynaptic action, whereas horizontal lines in the CV-2 plot indicate a change in postsynaptic responsiveness.

### Paired-pulse ratio (PPR)

In some experiments, every 10^{th} EPSP was replaced by two consecutive EPSPs (EPSP 1/2) with an interval of 50 ms, and their maximal amplitudes were measured. The PPR was then calculated as (EPSP2 (mV))/(EPSP 1 (mV)), and the mean PPR values were compared between baseline before induction of weak-aLTP and 25-30 minutes thereafter. A change in the PPR commonly indicates modification of presynaptic transmitter release probability, whereas no change indicates a postsynaptic mechanism.

### References:

Aartsma-Rus A, van Vliet L, Hirschi M, Janson AA, Heemskerk H, de Winter CL, de Kimpe S, van Deutekom JC, 't Hoen PA, van Ommen GJ. Guidelines for antisense oligonucleotide design and insight into splice-modulating mechanisms. Mol Ther. 2009 Mar;17(3):548-53. doi: 10.1038/mt.2008.205. Epub 2008 Sep 23. PMID: 18813282; PMCID: PMC2835096.
Alboni S, van Dijk RM, Poggini S, Milior G, Perrotta M, Drenth T, Brunello N, Wolfer DP, Lima-tola C, Amrein I, Cirulli F, Maggi L, Branchi I. Fluoxetine effects on molecular, cellular and behavioral endophenotypes of depression are driven by the living environment. Mol Psychiatry. 2017 Apr;22(4):552-561. doi: 10.1038/mp.2015.142. Epub 2015 Sep 15. Erratum in: Mol Psychiatry. 2017 Apr;22(4):635. PMID: 26645631; PMCID: PMC5378807.
Castrén E. Neuronal network plasticity and recovery from depression. JAMA Psychiatry. 2013 Sep;70(9):983-9. doi: 10.1001/jamapsychiatry.2013.1. PMID: 23842648.
Castrén E, Antila H. Neuronal plasticity and neurotrophic factors in drug responses. Mol Psychiatry. 2017 Aug;22(8):1085-1095. doi: 10.1038/mp.2017.61. Epub 2017 Apr 11. PMID: 28397840; PMCID: PMC5510719.
Costa BM, Feng B, Tsintsadze TS, Morley RM, Irvine MW, Tsintsadze V, Lozovaya NA, Jane DE, Monaghan DT. N-methyl-D-aspartate (NMDA) receptor NR2 subunit selectivity of a series of novel piperazine-2,3-dicarboxylate derivatives: preferential blockade of extrasynaptic NMDA receptors in the rat hippocampal CA3-CA1 synapse. J Pharmacol Exp Ther. 2009 Nov;331(2):618-26. doi: 10.1124/jpet.109.156752. Epub 2009 Aug 14. PMID: 19684252; PMCID: PMC2775268.
Gerhard DM, Pothula S, Liu RJ, Wu M, Li XY, Girgenti MJ, Taylor SR, Duman CH, Delpire E, Picciotto M, Wohleb ES, Duman RS. GABA interneurons are the cellular trigger for ketamine's rapid antidepressant actions. J Clin Invest. 2020 Mar 2;130(3):1336-1349. doi: 10.1172/JCI130808. PMID: 31743111; PMCID: PMC7269589.
Hansen KB, Traynelis SF. Structural and mechanistic determinants of a novel site for noncompetitive inhibition of GluN2D-containing NMDA receptors. J Neurosci. 2011 Mar 9;31(10):3650-61. doi: 10.1523/JNEUROSCI.5565-10.2011. PMID: 21389220; PMCID: PMC3063124.
Hansen KB, Wollmuth LP, Bowie D, Furukawa H, Menniti FS, Sobolevsky Al, Swanson GT, Swanger SA, Greger IH, Nakagawa T, McBain CJ, Jayaraman V, Low CM, Dell'Acqua ML, Diamond JS, Camp CR, Perszyk RE, Yuan H, Traynelis SF. Structure, Function, and Pharmacology of Glutamate Receptor Ion Channels. Pharmacol Rev. 2021 Oct;73(4):298-487. doi: 10.1124/pharmrev.120.000131. PMID: 34753794; PMCID: PMC8626789.
Holz A, Mülsch F, Schwarz MK, Hollmann M, Döbrössy MD, Coenen VA, Bartos M, Normann C, Biber K, van Calker D, Serchov T. Enhanced mGlu5 Signaling in Excitatory Neurons Promotes Rapid Antidepressant Effects via AMPA Receptor Activation. Neuron. 2019 Oct 23;104(2):338-352.e7. doi: 10.1016/j.neuron.2019.07.011. Epub 2019 Aug 13. PMID: 31420117.
Honoré T, Davies SN, Drejer J, Fletcher EJ, Jacobsen P, Lodge D, Nielsen FE. Quinoxalinedi-ones: potent competitive non-NMDA glutamate receptor antagonists. Science. 1988 Aug 5;241(4866):701-3. doi: 1 0.1126/science.2899909. PMID: 2899909.
Hu B, Zhong L, Weng Y, Peng L, Huang Y, Zhao Y, Liang XJ. Therapeutic siRNA: state of the art. Signal Transduct Target Ther. 2020 Jun 19;5(1):101. doi: 10.1038/s41392-020-0207-x. PMID: 32561705; PMCID: PMC7305320.
Ide S, Ikekubo Y, Mishina M, Hashimoto K, Ikeda K. Role of NMDA receptor GluN2D subunit in the antidepressant effects of enantiomers of ketamine. J Pharmacol Sci. 2017 Nov;135(3):138-140. doi: 10.1016/j.jphs.2017.11.001. Epub 2017 Nov 10. PMID: 29174627.
Kaila K. Ionic basis of GABAA receptor channel function in the nervous system. Prog Neurobiol. 1994 Mar;42(4):489-537. doi: 10.1016/0301-0082(94)90049-3. PMID: 7522334.
Khlestova E, Johnson JW, Krystal JH, Lisman J. The Role of GluN2C-Containing NMDA Receptors in Ketamine's Psychotogenic Action and in Schizophrenia Models. J Neurosci. 2016 Nov 2;36(44):11151-11157. doi: 10.1523/JNEUROSCI.1203-16.2016. PMID: 27807157; PMCID: PMC5148234.
Lodge D, Davies SN, Jones MG, Millar J, Manallack DT, Ornstein PL, Verberne AJ, Young N, Beart PM. A comparison between the in vivo and in vitro activity of five potent and competitive NMDA antagonists. Br J Pharmacol. 1988 Nov;95(3):957-65. doi: 10.1111/j.1476-5381.1988.tb11726.x. PMID: 2905186; PMCID: PMC1854208.
Monyer H, Burnashev N, Laurie DJ, Sakmann B, Seeburg PH. Developmental and regional expression in the rat brain and functional properties of four NMDA receptors. Neuron. 1994 Mar;12(3):529-40. doi: 10.1016/0896-6273(94)90210-0. PMID: 7512349.
Mosley CA, Acker TM, Hansen KB, Mullasseril P, Andersen KT, Le P, Vellano KM, Bräuner-Os-borne H, Liotta DC, Traynelis SF. Quinazolin-4-one derivatives: A novel class of noncompetitive NR2C/D subunit-selective N-methyl-D-aspartate receptor antagonists. J Med Chem. 2010 Aug 12;53(15):5476-90. doi: 10.1021/jm100027p. PMID: 20684595; PMCID: PMC2920070.
Naito Y, Ui-Tei K. siRNA Design Software for a Target Gene-Specific RNA Interference. Front Genet. 2012 Jun 11;3:102. doi: 10.3389/fgene.2012.00102. PMID: 22701467; PMCID: PMC3371628.
Njoo C, Heinl C, Kuner R. In vivo SiRNA transfection and gene knockdown in spinal cord via rapid noninvasive lumbar intrathecal injections in mice. J Vis Exp. 2014 Mar 22;(85):51229. doi: 10.3791/51229. PMID: 24686916; PMCID: PMC4155904.
Perszyk RE, DiRaddo JO, Strong KL, Low CM, Ogden KK, Khatri A, Vargish GA, Pelkey KA, Tricoire L, Liotta DC, Smith Y, McBain CJ, Traynelis SF. GluN2D-Containing N-methyl-d-Aspartate Receptors Mediate Synaptic Transmission in Hippocampal Interneurons and Regulate Interneuron Activity. Mol Pharmacol. 2016 Dec;90(6):689-702. doi: 10.1124/mol.116.105130. Epub 2016 Sep 13. PMID: 27625038; PMCID: PMC5118640.
Preskorn SH, Baker B, Kolluri S, Menniti FS, Krams M, Landen JW. An innovative design to establish proof of concept of the antidepressant effects of the NR2B subunit selective N-methyl-D-aspartate antagonist, CP-101,606, in patients with treatment-refractory major depressive disorder. J Clin Psychopharmacol. 2008 Dec;28(6):631-7. doi: 10.1097/JCP.0b013e31818a6cea. PMID: 19011431.
Rao DD, Vorhies JS, Senzer N, Nemunaitis J. siRNA vs. shRNA: similarities and differences. Adv Drug Deliv Rev. 2009 Jul 25;61(9):746-59. doi: 10.1016/j.addr.2009.04.004. Epub 2009 Apr 20. PMID: 19389436.
Ravikrishnan A, Gandhi PJ, Shelkar GP, Liu J, Pavuluri R, Dravid SM. Region-specific Expression of NMDA Receptor GluN2C Subunit in Parvalbumin-Positive Neurons and Astrocytes: Analysis of GluN2C Expression using a Novel Reporter Model. Neuroscience. 2018 Jun 1;380:49-62. doi: 10.1016/j.neuroscience.2018.03.011. Epub 2018 Mar 17. PMID: 29559384; PMCID: PMC6086378.
Serchov T, Clement HW, Schwarz MK, lasevoli F, Tosh DK, Idzko M, Jacobson KA, de Bartolo-meis A, Normann C, Biber K, van Calker D. Increased Signaling via Adenosine A1 Receptors, Sleep Deprivation, Imipramine, and Ketamine Inhibit Depressive-like Behavior via Induction of Homer1a. Neuron. 2015 Aug 5;87(3):549-62. doi: 10.1016/j.neuron.2015.07.010. PMID: 26247862; PMCID: PMC4803038.
Shu Y, Diamond TL, Hershey JC, Huang S, Magliaro BC, O'Brien JA, Schlegel KS, Puri V, Uebele VN, Uslaner JM, Wang C, Converso A. Discovery of 4-arylquinoline-2-carboxamides, highly potent and selective class of mGluR2 negative allosteric modulators: From HTS to activity in animal models. Bioorg Med Chem Lett. 2020 May 1;30(9):127066. doi: 10.1016/j.bmcl.2020.127066. Epub 2020 Feb 28. PMID: 32173198.
Swanger SA, Vance KM, Acker TM, Zimmerman SS, DiRaddo JO, Myers SJ, Bundgaard C, Mosley CA, Summer SL, Menaldino DS, Jensen HS, Liotta DC, Traynelis SF. A Novel Negative Allosteric Modulator Selective for GluN2C/2D-Containing NMDA Receptors Inhibits Synaptic Transmission in Hippocampal Interneurons. ACS Chem Neurosci. 2018 Feb 21;9(2):306-319. doi: 10.1021/acschemneuro.7b00329. Epub 2017 Nov 2. PMID: 29043770; PMCID: PMC5924706.
Traynelis SF, Wollmuth LP, McBain CJ, Menniti FS, Vance KM, Ogden KK, Hansen KB, Yuan H, Myers SJ, Dingledine R. Glutamate receptor ion channels: structure, regulation, and function. Pharmacol Rev. 2010 Sep;62(3):405-96. doi: 10.1124/pr.109.002451. Erratum in: Pharmacol Rev. 2014 Oct;66(4):1141. PMID: 20716669; PMCID: PMC2964903.
Vestring S, Serchov T, Normann C. Animal Models of Depression - Chronic Despair Model (CDM). J Vis Exp. 2021 Sep 23;(175). doi: 10.3791/62579. PMID: 34633373.
Vyklicky V, Korinek M, Smejkalova T, Balik A, Krausova B, Kaniakova M, Lichnerova K, Cerny J, Krusek J, Dittert I, Horak M, Vyklicky L. Structure, function, and pharmacology of NMDA receptor channels. Physiol Res. 2014;63(Suppl 1):S191-203. doi: 10.33549/physiolres.932678. PMID: 24564659.
Widman AJ, McMahon LL. Disinhibition of CA1 pyramidal cells by low-dose ketamine and other antagonists with rapid antidepressant efficacy. Proc Natl Acad Sci U S A. 2018 Mar 27;115(13):E3007-E3016. doi: 10.1073/pnas.1718883115. Epub 2018 Mar 12. PMID: 29531088; PMCID: PMC5879689.
Yao L, Grand T, Hanson JE, Paoletti P, Zhou Q. Higher ambient synaptic glutamate at inhibitory versus excitatory neurons differentially impacts NMDA receptor activity. Nat Commun. 2018 Oct 1;9(1):4000. doi: 10.1038/s41467-018-06512-7. Erratum in: Nat Commun. 2018 Nov 15;9(1):4887. PMID: 30275542; PMCID: PMC6167324.
Zanos P, Highland JN, Stewart BW, Georgiou P, Jenne CE, Lovett J, Morris PJ, Thomas CJ, Moaddel R, Zarate CA Jr, Gould TD. (2R,6R)-hydroxynorketamine exerts mGlu2 receptor-dependent antidepressant actions. Proc Natl Acad Sci U S A. 2019 Mar 26;116(13):6441-6450. doi: 10.1073/pnas.1819540116. Epub 2019 Mar 13. PMID: 30867285; PMCID: PMC6442605.
Zhang B, Yang X, Ye L, Liu R, Ye B, Du W, Shen F, Li Q, Guo F, Liu J, Guo F, Li Y, Xu Z, Liu Z. Ketamine activated glutamatergic neurotransmission by GABAergic disinhibition in the medial prefrontal cortex. Neuropharmacology. 2021 Aug 15;194:108382. doi: 10.1016/j.neuro-pharm.2020.108382. Epub 2020 Nov 2. PMID: 33144117.

## Claims

1. A GluN2D inhibitor for use in the relapse prevention or treatment of a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34, , wherein the GluN2D inhibitor is not *R*,*S*-ketamine or a pharmaceutically acceptable salt thereof, *S*-ketamine or a pharmaceutically acceptable salt thereof, or R-ketamine or a pharmaceutically acceptable salt thereof.

2. The GluN2D inhibitor for use according to claim 1, wherein the GluN2D inhibitor causes disinhibition and/or increases synaptic plasticity.

3. The GluN2D inhibitor for use according to claim 1 or 2, wherein the GluN2D inhibitor is selected from the group consisting of a small molecule targeting GluN2D, an antibody directed to GluN2D or an antigen-binding fragment thereof, an antisense oligonucleotide targeting the GluN2D mRNA, a small interfering RNA (siRNA) targeting the GluN2D mRNA, a short hairpin RNA (shRNA) targeting the GluN2D mRNA, a microRNA (miRNA) targeting the GluN2D mRNA and a CRISPR-guide RNA (sgRNA) targeting GluN2D gene transcription.

4. The GluN2D inhibitor for use according to claim 3, wherein the GluN2D inhibitor is an antisense oligonucleotide targeting the GluN2D mRNA, which is capable of binding to and/or is at least partially complementary to a region of the GluN2D gene or a regulatory element thereof.

5. The GluN2D inhibitor for use according to claim 3, wherein the GluN2D inhibitor is a siRNA targeting the GluN2D mRNA, which is capable of interfering with the gene expression of the GluN2D gene and comprises a first strand at least partially complementary to 15 nucleotides of the GluN2D gene, and a second strand of 15 to 30 nucleotides in length, wherein at least 12 nucleotides of the first strand and second strand are complementary to each other and form a siRNA duplex.

6. The GluN2D inhibitor for use according to claim 3, wherein the GluN2D inhibitor is a sgRNA targeting GluN2D gene transcription, which is at least partially complementary to 15 nucleotides of the GluN2D gene or its upstream regulatory elements, and wherein in addition a CRISPR protein lacking endonuclease activity is administered, preferably the CRISPR protein is fused to at least a domain of Krüppel associated box (KRAB) protein.

7. A vector encoding a GluN2D inhibitor selected from the group consisting of an antisense oligonucleotide targeting the GluN2D mRNA, a siRNA targeting the GluN2D mRNA, a shRNA targeting the GluN2D mRNA, a miRNA targeting the GluN2D mRNA and a sgRNA targeting GluN2D gene transcription for use in the treatment or relapse prevention of a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34.

8. A pharmaceutical composition comprising a GluN2D inhibitor, wherein the GluN2D inhibitor is not *R*,*S*-ketamine or a pharmaceutically acceptable salt thereof, *S*-ketamine or a pharmaceutically acceptable salt thereof, or R-ketamine or a pharmaceutically acceptable salt thereof, and a pharmaceutically acceptable excipient for use in the treatment or relapse prevention of a depressive episode occurring (i) in isolation as defined in ICD-10: F32; (ii) as part of a bipolar affective disorder as defined in ICD-10: F31; (iii) as part of a recurrent depressive disorder as defined in ICD-10: F33; or (iv) as part of a persistent mood disorder as defined in ICD-10: F34.

9. The pharmaceutical composition for use according to claim 8, wherein the GluN2D inhibitor is selected from the group consisting of an antisense oligonucleotide targeting the GluN2D mRNA, a siRNA targeting the GluN2D mRNA, a shRNA targeting the GluN2D mRNA, a miRNA targeting the GluN2D mRNA and a sgRNA targeting GluN2D gene transcription; and the pharmaceutically acceptable excipient comprises a lipid.

## Patentansprüche

1. GluN2D-Inhibitor zur Verwendung bei der Rückfallprävention oder Behandlung einer depressiven Episode, die auftritt (i) isoliert, wie in ICD-10: F32 definiert; (ii) im Rahmen einer bipolaren affektiven Störung, wie in ICD-10: F31 definiert; (iii) im Rahmen einer rezidivierenden depressiven Störung, wie in ICD-10: F33 definiert; oder (iv) im Rahmen einer anhaltenden affektiven Störung, wie in ICD-10: F34 definiert, wobei der GluN2D-Inhibitor nicht R,S-Ketamin oder ein pharmazeutisch akzeptables Salz davon, S-Ketamin oder ein pharmazeutisch akzeptables Salz davon oder R-Ketamin oder ein pharmazeutisch akzeptables Salz davon ist.

2. GIuN2D-Inhibitor zur Verwendung nach Anspruch 1, wobei der GluN2D-Inhibitor Disinhibition bewirkt und/oder die synaptische Plastizität erhöht.

3. GluN2D-Inhibitor zur Verwendung nach Anspruch 1 oder 2, wobei der GluN2D-Inhibitor ausgewählt ist aus der Gruppe bestehend aus einem kleinen Molekül, das auf GluN2D abzielt, einem Antikörper, der auf GluN2D ausgerichtet ist, oder einem antigenbindenden Fragment davon, einem Antisense-Oligonucleotid, das auf die GluN2D-mRNA abzielt, einer kleinen interferierenden RNA (siRNA), die auf die GluN2D-mRNA abzielt, einer kurzen Haarnadel-RNA (shRNA), die auf die GluN2D-mRNA abzielt, einer Mikro-RNA (miRNA), die auf die GluN2D-mRNA abzielt, und einer CRISPR-Leit-RNA (sgRNA), die auf die Transkription des GluN2D-Gens abzielt.

4. GIuN2D-Inhibitor zur Verwendung nach Anspruch 3, wobei der GluN2D-Inhibitor ein auf die GluN2D-mRNA abzielendes Antisense-Oligonucleotid ist, das imstande ist, an eine Region des GluN2D-Gens oder ein regulatorisches Element davon zu binden und/oder zumindest teilweise komplementär dazu ist.

5. GluN2D-Inhibitor zur Verwendung nach Anspruch 3, wobei der GluN2D-Inhibitor eine auf die GluN2D-mRNA abzielende siRNA ist, die imstande ist, die Genexpression des GluN2D-Gens zu stören, und die einen ersten Strang, der zumindest teilweise komplementär zu 15 Nucleotiden des GluN2D-Gens ist, und einen zweiten Strang von 15 bis 30 Nucleotiden in der Länge umfasst, wobei mindestens 12 Nucleotide des ersten Strangs und zweiten Strangs komplementär zueinander sind und einen siRNA-Doppelstrang bilden.

6. GluN2D-Inhibitor zur Verwendung nach Anspruch 3, wobei der GluN2D-Inhibitor eine auf die Transkription des GluN2D-Gens abzielende sgRNA ist, die zumindest teilweise komplementär zu 15 Nucleotiden des GluN2D-Gens oder seinen stromaufwärts gelegenen regulatorischen Elementen ist, und wobei außerdem ein CRISPR-Protein ohne Endonuclease-Aktivität verabreicht wird, vorzugsweise ist das CRISPR-Protein an mindestens eine Domäne eines (Krüppel-assoziierte Box)-Proteins (KRAB-Proteins) fusioniert.

7. Vektor, der einen GluN2D-Inhibitor, ausgewählt aus der Gruppe bestehend aus einem Antisense-Oligonucleotid, das auf die GluN2D-mRNA abzielt, einer siRNA, die auf die GluN2D-mRNA abzielt, einer shRNA, die auf die GluN2D-mRNA abzielt, einer miRNA, die auf die GluN2D-mRNA abzielt, und einer sgRNA, die auf die Transkription des GluN2D-Gens abzielt, codiert, zur Verwendung bei der Behandlung oder Rückfallprävention einer depressiven Episode, die auftritt (i) isoliert, wie in ICD-10: F32 definiert; (ii) im Rahmen einer bipolaren affektiven Störung, wie in ICD-10: F31 definiert; (iii) im Rahmen einer rezidivierenden depressiven Störung, wie in ICD-10: F33 definiert; oder (iv) im Rahmen einer anhaltenden affektiven Störung, wie in ICD-10: F34 definiert.

8. Pharmazeutische Zusammensetzung, umfassend einen GluN2D-Inhibitor, wobei der GluN2D-Inhibitor nicht R,S-Ketamin oder ein pharmazeutisch akzeptables Salz davon, S-Ketamin oder ein pharmazeutisch akzeptables Salz davon oder R-Ketamin oder ein pharmazeutisch akzeptables Salz davon ist, und einen pharmazeutisch akzeptablen Exzipienten, zur Verwendung bei der Behandlung oder Rückfallprävention einer depressiven Episode, die auftritt (i) isoliert, wie in ICD-10: F32 definiert; (ii) im Rahmen einer bipolaren affektiven Störung, wie in ICD-10: F31 definiert; (iii) im Rahmen einer rezidivierenden depressiven Störung, wie in ICD-10: F33 definiert; oder (iv) im Rahmen einer anhaltenden affektiven Störung, wie in ICD-10: F34 definiert.

9. Pharmazeutische Zusammensetzung zur Verwendung nach Anspruch 8, wobei der GluN2D-Inhibitor ausgewählt ist aus der Gruppe bestehend aus einem Antisense-Oligonucleotid, das auf die GluN2D-mRNA abzielt, einer siRNA, die auf die GluN2D-mRNA abzielt, einer shRNA, die auf die GluN2D-mRNA abzielt, einer miRNA, die auf die GluN2D-mRNA abzielt, und einer sgRNA, die auf die Transkription des GluN2D-Gens abzielt; und der pharmazeutisch akzeptable Exzipient ein Lipid umfasst.

## Revendications

1. Inhibiteur de GluN2D pour utilisation dans la prévention des rechutes ou le traitement d'un épisode dépressif survenant (i) de manière isolée tel que défini dans la CIM-10 : F32 ; (ii) dans le cadre d'un trouble affectif bipolaire tel que défini dans la CIM-10 : F31 ; (iii) dans le cadre d'un trouble dépressif récurrent tel que défini dans la CIM-10 : F33 ; ou (iv) dans le cadre d'un trouble de l'humeur persistant tel que défini dans la CIM-10 : F34, l'inhibiteur de GluN2D n'étant pas la R,S-kétamine ou un sel pharmaceutiquement acceptable de celle-ci, la S-kétamine ou un sel pharmaceutiquement acceptable de celle-ci, ou la R-kétamine ou un sel pharmaceutiquement acceptable de celle-ci.

2. Inhibiteur de GluN2D pour utilisation selon la revendication 1, l'inhibiteur de GluN2D provoquant une désinhibition et/ou augmentant la plasticité synaptique.

3. Inhibiteur de GluN2D pour utilisation selon la revendication 1 ou 2, l'inhibiteur de GluN2D étant choisi dans le groupe constitué par une petite molécule ciblant GluN2D, un anticorps dirigé contre GluN2D ou un fragment de liaison à l'antigène de celui-ci, un oligonucléotide antisens ciblant l'ARNm de GluN2D, un petit ARN interférent (siRNA) ciblant l'ARNm de GluN2D, un ARN en épingle à cheveux court (shRNA) ciblant l'ARNm de GluN2D, un microARN (miRNA) ciblant l'ARNm de GluN2D, et un ARN guide CRISPR (sgRNA) ciblant la transcription du gène GluN2D.

4. Inhibiteur de GluN2D pour utilisation selon la revendication 3, l'inhibiteur de GluN2D étant un oligonucléotide antisens ciblant l'ARNm de GluN2D, qui est capable de se lier à et/ou est au moins partiellement complémentaire d'une région du gène GluN2D ou d'un élément régulateur de celui-ci.

5. Inhibiteur de GluN2D pour utilisation selon la revendication 3, l'inhibiteur de GluN2D étant un siRNA ciblant l'ARNm de GluN2D, qui est capable d'interférer avec l'expression génique du gène GluN2D et comprend un premier brin au moins partiellement complémentaire de 15 nucléotides du gène GluN2D, et un deuxième brin de 15 à 30 nucléotides de longueur, au moins 12 nucléotides du premier brin et du deuxième brin étant complémentaires les uns des autres et formant un duplex de siRNA.

6. Inhibiteur de GluN2D pour utilisation selon la revendication 3, l'inhibiteur de GluN2D étant un sgRNA ciblant la transcription du gène GluN2D, qui est au moins partiellement complémentaire de 15 nucléotides du gène GluN2D ou de ses éléments régulateurs en amont, et en outre une protéine CRISPR dépourvue d'activité endonucléase étant administrée, de préférence la protéine CRISPR étant fusionnée à au moins un domaine de la protéine Krüppel associated box (KRAB).

7. Vecteur codant pour un inhibiteur de GluN2D choisi dans le groupe constitué par un oligonucléotide antisens ciblant l'ARNm de GluN2D, un siRNA ciblant l'ARNm de GluN2D, un shRNA ciblant l'ARNm de GluN2D, un miRNA ciblant l'ARNm de GluN2D et un sgRNA ciblant la transcription du gène GluN2D pour utilisation dans le traitement ou la prévention des rechutes d'un épisode dépressif survenant (i) de manière isolée tel que défini dans la CIM-10 : F32 ; (ii) dans le cadre d'un trouble affectif bipolaire tel que défini dans la CIM-10 : F31 ; (iii) dans le cadre d'un trouble dépressif récurrent tel que défini dans la CIM-10 : F33 ; ou (iv) dans le cadre d'un trouble de l'humeur persistant tel que défini dans la CIM-10 : F34.

8. Composition pharmaceutique comprenant un inhibiteur de GluN2D, l'inhibiteur de GluN2D n'étant pas de la R,S-kétamine ou un sel pharmaceutiquement acceptable de celle-ci, la S-kétamine ou un sel pharmaceutiquement acceptable de celle-ci, ou la R-kétamine ou un sel pharmaceutiquement acceptable de celle-ci, et un excipient pharmaceutiquement acceptable, pour utilisation dans le traitement ou la prévention des rechutes d'un épisode dépressif survenant (i) de manière isolée tel que défini dans la CIM-10 : F32 ; (ii) dans le cadre d'un trouble affectif bipolaire tel que défini dans la CIM-10 : F31 ; (iii) dans le cadre d'un trouble dépressif récurrent tel que défini dans la CIM-10 : F33 ; ou (iv) dans le cadre d'un trouble de l'humeur persistant tel que défini dans la CIM-10 : F34.

9. Composition pharmaceutique pour utilisation selon la revendication 8, l'inhibiteur de GluN2D étant choisi dans le groupe constitué par un oligonucléotide antisens ciblant l'ARNm de GluN2D, un siRNA ciblant l'ARNm de GluN2D, un shRNA ciblant l'ARNm de GluN2D, un miRNA ciblant l'ARNm de GluN2D et un sgRNA ciblant la transcription du gène GluN2D ; et l'excipient pharmaceutiquement acceptable comprenant un lipide.
